# EUROPEAN PATENT APPLICATION

(11) **EP 3 153 156 A1**
(43) Date of publication of application: **12.04.2017**
(21) Application number: 15799012.8
(22) Date of filing: 02.06.2015
(51) Int. Cl.: A61K 9/08, A61K 31/728, A61K 31/14, A61K 47/02

(54) **COMPOSITION BASED ON A STABILIZED SOLUTION OF ACTIVE INGREDIENTS**

(30) Priority: 30.05.2014 UA 201405946; 03.06.2014 UA 201406108 U; 29.05.2015 UA 201505330; 29.05.2015 UA 201505327
(71) Applicant: Derkach, Nataliia Mykolaivna, Kiev 03110 (UA)
(72) Inventor: Derkach, Nataliia Mykolaivna, Kiev 03110 (UA)
(74) Representative: Anohins, Vladimirs
(86) International application number: PCT/UA2015/000047
(87) International publication number: WO 2015/183230

(57) **Abstract**

This invention relates to pharmaceutical compositions (variants) based on a stabilized solution of two active ingredients, said stabilized solution of two active ingredients comprising, as first active ingredient, low-molecular weight hyaluronic acid and/or a pharmaceutically acceptable salt thereof, wherein the molecular mass of said acid or salt is from 100 kDa to 400 kDa or, in a variant, from 400 kDa to 2.5 MDa, and, as second active ingredient, Decametoxin or a water-soluble salt thereof, and a stabilizer in the form of a pharmaceutically acceptable salt that is capable of dissolving in water and dissociating in aqueous solutions into metal cations and acid residue anions, or a mixture of such salts, wherein the stabilizer is capable of forming water-soluble compounds with hyaluronic acid and/or a pharmaceutically acceptable salt thereof and Decametoxin or a water-soluble salt thereof, simultaneously. The stabilized solution further contains at least one pharmaceutically acceptable additive.

## Description

### FIELD OF INVENTION

Technical solution belongs to the field of medicine, in particular to compositions which possess anti-inflammatory, antiseptic, regenerating and moistening action and may be used in the treatment of skin and organs lesions, and in particular possess therapeutic activity in regard to suppression of adhesive process.

### PRIOR ART

Adhesions are pathological fusions of tissues, which are formed by fragments of scar tissue and appear in non-typical places due to injuries caused by surgical trauma, infection, ischemia or action of foreign materials. The adhesions may be thin strips of tissue similar to polyethylene film, or thick filamentous bands. The process of adhesions formation is called adhesive process.

Adhesions are formed as a result of inflammatory process which may arise in internal organs diseases, after surgery, in infections and traumas. Although adhesions may form between different organs, most often they arise between intestinal loops, uterine tubes, ovaries, uterus, urinary bladder and heart.

Adhesions in abdominal cavity are widespread complication after surgical operation, which is observed in 93% of people which underwent surgery of pelvic organs or abdominal cavity. Abdominal adhesions are also observed in 10.4% of people who never had surgery.

In most cases adhesions are painless and cause no complications. Nevertheless, adhesions account for 60-70% of small bowel obstruction cases in adults and promote chronic pain development in pelvic region.

Adhesions usually begin to form in the first few days after operation, but they may produce no symptoms over a period of several months or even years. As the scar tissue begins to restrict movement in small intestine, the food movement in digestive system gets more complicated, and probability of intestinal obstruction development arises.

In extreme cases, adhesions may form fibrous bands around bowel segments. It limits blood supply and leads to atrophy of tissues.

Scar tissue may form within connective-tissue heart sac (pericardium), thus hindering its normal operation. Various infections, such as rheumatism, lead to adhesions formation on heart valves and decrease in its functional efficiency.

Adhesions in small pelvis may form in small pelvis organs, such as uterus, ovaries, uterine tubes or urinary bladder. As a rule, they arise after operation. Inflammatory diseases of pelvic organs and complications after infections (for example, venereal diseases) often leads to adhesions formation in uterine tubes through which ova travel into the uterus for fertilization. Adhesions of uterine tubes may result in infertility and increase the risk of ectopic pregnancy.

Very often, adhesive process after gynaecological operations has no clinical implications altogether, however, the presence of adhesions should be accounted for due to possibility of early or delayed complications, including infertility, pelvic pain, and intestinal obstruction, accompanied by decrease in quality of life. This often requires readmission of patient and additional more complex surgical intervention, which increases substantially cost of the treatment.

Disposition to adhesions formation is an individual feature of each person. Different factors, such as diet, chronic diseases, diabetes mellitus, and chronic infectious processes, impair functions of leukocytes and fibrinolytic activity, and potentially enhance adhesion formation. It is also proven that incidence of postoperative adhesions development increases with patient age, number of previous operations and volume of surgical intervention. Any operation in abdominal cavity may result in adhesion formation even at minimal damage to serous membrane, leading to conglutination of almost all structures. It has been established that adhesions are formed in 60-90% of women undergoing radical gynaecological operations. Some studies indicate that women who had at least one operation are subjected to repeat operation with probability of 5%. Such women have greater probability of hospitalization owing to adhesive disease during the following 10 years. Threfore, adhesions are cause of hospitalization in 20% of women patients.

Need in decreasing rate of postoperative adhesion development is obvious. For example, more than 440,000 operations of abdominal and pelvic adhesiolysis are performed in the USA annually, thus creating additional risks for patients.

In search of effective methods of prophylaxis of adhesions, a lot of clinical techniques and anti-adhesion preparations have been proposed, which should prevent the formation of primary and secondary postoperative adhesions. Principal approach to prophylaxis of adhesions includes corresponding surgical technique with limited trauma to intraabdominal structures and use of anti-adhesion preparations reducing adhesion formation.

Anti-adhesion preparations may be directed against different factors of the inflammatory process (for example, infections, endotoxins, exudate) and/or adhesions formation (for example, hemocoagulation, fibrin accumulation, fibroblastic activity and proliferation). Any anti-adhesion preparation should act specifically against factors of the adhesive process, but not against the process of normal wound healing.

In literature there are described studies on influencing the adhesive process with anti-adhesion preparations belonging to the following classes: non-steroidal anti-inflammatory drugs, glucocorticoid and antihistamine preparations, progesterone/oestrogen preparations, anticoagulants, fibrinolytic preparations, antibiotics. Data of clinical trials and animal studies demonstrate that said anti-adhesion preparations do not solve the problem of postoperative adhesions formation - their use is limited by their insufficient efficiency, and also in some cases - by their insufficient safety (presence of unwanted side effects), presence of inflammatory processes, probably appearance of infections which leads to side effects and retardation of wound healing.

Therefore the authors have proposed stabilized solution and pharmaceutical compositions on its basis, which can display anti-adhesive action, and also substances of pharmaceutical compositions providing both wound healing and anti-inflammatory disinfecting action. Hyaluronic acid and/or its pharmaceutically acceptable salts are selected as substance possessing anti-adhesive action.

Hyaluronic acid (HA) is a natural glycosaminoglycan, which is a main component of extracellular substance of connective tissue. HA is a biocompatible, nonimmunogenic, nontoxic and naturally bioabsorbing substance. It has negative charge at physiological pH and is readily soluble in water, natural HA has molecular weight from 5 thousand to 20 million Da. Average molecular weight of HA in synovial liquid of a man is about 3 million Da. HA in the tissues of serous membrane provides certain protection from drying and other types of serous membrane damage. HA is also found in skin, where it participates in tissue regeneration process. Excessive ultraviolet radiation exposure of skin causes its inflammation ("solar burn"), with HA synthesis ceasing in dermal cells and its decomposition rate increasing.

There is known a preparation for prophylaxis of adhesion formation named Separacoat (manufactured by Genzyme, USA), which is a 0,04% HA solution in conjunction with phosphate buffer (Matveev N.L., Arutyunyan D.Yu., Vnutribryushnye spaiki - nedootsenivaemaya problema (Intraabdominal adhesions - unappreciated problem). - Endoskopicheskaya Khirurgiya (Endoscopic surgery).-2007.-No 5.-p.60-69). It had been used intraoperatively, to dissection, to protect abdominal surfaces against indirect surgical trauma, rather than postoperatively, for separation of surfaces after their traumatization. The studies have demonstrated the inefficiency of this preparation. It was not approved by the Food and Drug Administration (FDA) on basis of the results of clinical trials and its production has been discontinued.

There is known a preparation Intergel for prevention of adhesions (Intergel solution, manufactured by Lifecore Biomedical, Inc, Chaska, MN) containing 5% solution of ferric hyaluronate. It had been demonstrated in previous studies that it decreases the number, severity and incidence of adhesions in abdominal surgery, however, use of this preparation in surgical operations involving dissection of intestinal lumen, resulted in unacceptably high rate of postoperative complications. Since 2003, the preparation has been withdrawn from clinical use due to cases of late postoperative pain in patients.

There is known from patent RU 2233164 (published 27.07.2004) a method for prophylaxis of postoperative abdominal adhesions development in abdominal operations, comprising application on the surgical wound of a film based on chemically modified hyaluronic acid, namely, hyaluronic acid, chemically bonded with 5-aminosalycilic acid.

However, when using hyaluronic acid-based preparations, a requirement arises in anti-inflammatory, antimicrobial and wound-healing action, which would substantially expand their applications and ease of their use in a wide range of inflammations and wounds, especially in operations, vaginal infections and birth activity and the like. for this purpose, hyaluronic acid-based preparation was used together with preparations providing anti-inflammatory wound-healing action. The attempts to develop single hyaluronic acid-based preparation which would provide also anti-inflammatory, wound-healing action, have led to the creation of patents that are described below.

To the anti-inflammatory preparations comprising hyaluronic acid RU 2438697 (published 10.01.2012) belong complex antiviral preparation for external use in treatment of herpes viral infections, containing recombinant interferon, hyaluronic acid, chitosan succinate, licorice extract, zinc oxide, lidocaine or dicain, polyethylene oxide, lanolin anhydrous, petrolatum, glycerol, and distilled water. Preparation provides prolonged anti-inflammatory, anesthetic, wound healing action in herpetic lesions of mucous membranes and skin, including genital herpes, ophthalmic herpes and tinea.

From patent application RU 2003101393 (published 27.06.2004), there is known a pharmaceutical composition for treatment of mucositis, stomatitis, and Behçet's syndrome, containing as active ingredients effective dosages of hyaluronic acid, glycyrrhetinic acid and polyvinylpyrrolidone in admixture with excipients and adjuvants suitable for local administration, and additionally containing antibacterial agents/disinfectants, and use of such composition in manufacturing of drugs for local treatment of inflammatory states of oral mucosa and oropharyngeal and oesophagal lining, in particular, mucisitis and stomatitis, and also vaginal and rectal mucosa (including, but not limited to, vestibulitis and Behçet's syndrome).

From patent application RU 2195262 (published 27.12.2002) there is known a pharmacological drug on base of hyaluronic acid possessing antimicrobial, wound-healing and anti-inflammatory action. The drug comprises hyaluronic acid, trimecaine, and polyethylene oxide, is a colorless homogeneous gelatinous mass, readily soluble in physiological saline and in water. Pharmacological drug is used in the treatment of infectious inflammatory diseases, including local and general putrid inflammatory processes, thermal and chemical burns, trophic ulcers in chronic venous failure, radiation injuries of skin, fissures, abrasions, and also in the treatment of wounds of different etiology.

From patent application RU 2501566 (published 20.12.2013) there is known a pharmaceutical composition for prophylaxis of postoperative scars development, of adhesions and keloids owing to surgical interference, including biologically active peptide containing amino acid sequence SEQ ID NO: 56, and high-molecular hyaluronic acid or its pharmaceutically acceptable salt.

Decamethoxine had been chosen as a substance possessing anti-inflammatory, antimicrobial, and bactericidal action.

Decamethoxine is a [1,10-decamethylene-bis(N,N-dimethylmethoxycarbonylmethyl)ammonium dichloride], it displays bactericidal activity in relation to gram-positive and gram-negative microorganisms. This compound demonstrates distinct bactericidal action on staphylococci, streptococci, blue pus bacillus, encapsulated bacteria, and fungicidal action on yeasts, yeast-like fungi, aspergilli, molds. Decamethoxine also enhances antibiotic sensitivity of bacteria.

Decamethoxine is known to be used as a solution locally and endobronchially in suppurative and fungal skin lesions (abscesses, suppurative wounds, candidiasis et al.), in proctitis, suppurative conjunctivitides, gingivitises, periodontitises, tonsillitides, otitises and other suppurative processes. It is prescribed endobronchially in lung diseases.

Besides, decamethoxine is used as antiseptic agent for hands treatment by medical staff and surgical field, and also to disinfect sutural and surgical materials, rubber gloves, medical instruments, devices and equipment made of metal, glass, plastic, and rubber, chemical sterilization and preservation of bone-and-sinew tyransplants.

Decamethoxine is a surface-active substance (SAS) acting by damaging cell wall integrity. The cell walls of microorganisms consist of short lipid chains which are destroyed rapidly by decamethoxine. The cell walls of human cells consist of long lipid chains, on which decamethoxine molecule has no effect.

Previously to the technical solution presented herein it was impossible to use preparations containing hyaluronic acid with decamethoxine, because decamethoxine is a cationic SAS and therefore is incompatible with soap and other anionic compounds, in particular, in solution with hyaluronic acid, because decamethoxine due to quaternary amino groups has positive charge, while hyaluronic acid is negatively charged due to carboxyl groups, and water insoluble precipitate is formed on their interaction in solution. This was the main problem not allowing joint use in a single preparation of these 2 substances, which in combination may provide effective treatment of adhesive processes, vaginal infections, and accomplish effective wound healing and anti-inflammatory action.

Even user instructions for drugs containing hyaluronic acid often mention the impossibility of their joint administration with quaternary amines, to which decamethoxine belongs. Therefore, there is no information in the literature concerning use of therapeutic agents containing simultaneously decamethoxine and hyaluronic acid in solution.

### Substance of the technical solution

The objective of the present technical solution is to provide a stabilized solution, which contains hyaluronic acid and/or its pharmaceutically acceptable salts and decamethoxine and/or its water soluble salts (hereinafter decamethoxine) and is stable in time and may be used in manufacture of preparations with a wide range of action in liquid or soft form, and a method for preparing of such stabilized solution.

The objective of the technical solution is to provide a novel pharmaceutical composition with a wide range of action comprizing stabilized solution of hyaluronic acid with decamethoxine.

One more objective of the present technical solution is to provide a novel pharmaceutical composition combining possibility of its use as a drug with therapeutic activity, which also possess anti-inflammatory, antiseptic, regenerating, and moistening action.

Another objective of the present technical solution is to provide enhanced efficiency of prophylaxis and therapy of adhesive disease and its consequences, increased quality of life after being subjected to surgery, suppression of the adhesive process, avoidance of organs dehydration with simultaneous anti-inflammatory and wound-healing action. As a result, wounds are healing faster, without adhesive processes, and without exacerbation of inflammatory processes.

One more objective is increased efficiency of vaginal infections treatment, to destroy pathogenic flora in gynaecology, stomatology, urology, proctology, ophthalmology, dermatovenereology, at that anti-inflammatory action is prolonged, with additional effect of protecting the mucous membranes against dehydration, their moistening, promoting their regeneration, decrease in the number of cases of soft tissues traumatism, in particular, genital tracts of women in childbirth, reduction in the length of labor and decrease in incidence of operative delivery, and also in neck surgery, when there exists a high risk of scar formation throughout all layers of the neck.

Also an objective is enhanced efficiency of wound healing process with simultaneous bactericidal, antimicrobial action.

Another objective is extending the range of compositions in pharmaceutically acceptable dosage form on base of said stabilized solution.

One of the objectives stated is achieved by providing stabilized solution on base of hyaluronic acid and/or its pharmaceutically acceptable salts (hereinafter HA) and decamethoxine by choice of concentrations and stabilizers and providing special conditions in the method for preparing of stabilized solution and pharmaceutical compositions on its basis in liquid or soft form, or in the form without precipitation in long-term stability form. Moreover, solution contains both HA and decamethoxine in liquid form and is stable in time.

As a result of studies it has been found unexpectedly that solution on base of decamethoxine and HA may be obtained by introducing stabilizer.

Stabilizer is a pharmaceutically acceptable salt capable of dissolving in water and dissotiating in aqueous solutions into metal cations and acid residues anions.

Mechanism of stabilization probably consists in shielding of carboxyl groups of hyaluronic acid and amino groups of decamethoxine by cations and anions of stabilizer, correspondingly.

Effective stabilizers, in particular, are nontoxic and low-toxic salts of inorganic acids, salts of organic mono- and dicarboxylic acids and the like.

Some specific stabilizers are sodium chloride, sodium succinate, sodium lactate, zinc chloride, zinc sulphate.

Thus, the objective of the invention is stabilized solution on basis of two active substances possessing therapeutic activity, characterized in that it further contains stabilizer, hyaluronic acid and/or its pharmaceutically acceptable salt is used as first active substance, decamethoxine and/or its water-soluble salt is used as second active substance, the stabilizer is a pharmaceutically acceptable salt, capable of dissolving in water and dissotiating in aqueous solutions into metal cations and acid residues anions, or mixture of such salts, said stabilizer being capable to form water-soluble compounds at the same time with hyaluronic acid and/or its pharmaceutically acceptable salt and with decamethoxine and/or its water-soluble salt.

Preferably, stabilizer is a nontoxic or low-toxic salt of inorganic acids, organic mono- and dicarboxylic acids, or mixture of such salts, in particular, stabilizer may be sodium chloride, sodium succinate, sodium lactate, zinc chloride, zinc sulphate or any mixture thereof.

Optionally, stabilized solution may further contain other pharmaceutically acceptable additives, in particular, to maintain pH of the solution at a constant level, for example, organic mono- or dicarboxylic acids, inorganic acids, alkalis, specifically, may contain an organic acid forming a salt, which is used therein as stabilizer, in particular for pH adjustment may be used a substance selected from the series of hydrochloric acid, sulfuric acid, nitric acid, phosphoric acid, acetic acid, propionic acid, hydroxyacetic acid, lactic acid, pyruvic acid, oxalic acid, malonic acid, succinic acid, maleic acid, fumaric acid, tartaric acid, citric acid, ascorbic acid, ammonium acetate, ammonia solution, alkaline-earth metal hydroxide, alkali metal hydroxide, sodium carbonate, sodium hydrocarbonate, sodium phosphate.

Preferably, content of the components may be as presented in the table below, mg/ml

| | |
|---|---|
| hyaluronic acid and/or its pharmaceutically acceptable salt | 1-50 |
| decamethoxine and/or its water-soluble salt | 0.01-10 |
| stabilizer | 1-60 |
| water | q.s. to 1 ml |

The stabilized solution may also contain pharmaceutically acceptable additive in the range of 1 to 60 mg/ml.

Preferably, the stabilized solution contains sodium succinate as stabilizer, and additionally succinic acid.

Preferably, the stabilized solution contains sodium lactate as stabilizer, and additionally lactic acid.

Also the stabilized solution may have the following composition, mg/ml:

| | |
|---|---|
| hyaluronic acid and/or its pharmaceutically acceptable salt | 5 |
| sodium succinate | 16 |
| succinic acid | 0.05 |
| sodium chloride | 1.2 |
| decamethoxine and/or its water-soluble salt | 0.2. |
| water | q.s. to 1 ml |

Another subject of the invention is a method for preparing of stabilized solution, wherein one of active substances and stabilizer are dissolved in water, and then second active substance is dissolved with mixing in the solution obtained to its complete dissolution, hyaluronic acid and/or its pharmaceutically acceptable salt and decamethoxine and/or its water-soluble salt being used as active substances, and stabilizer being a pharmaceutically acceptable salt, capable of dissolving in water and dissotiating in aqueous solutions into metal cations and acid residues anions, or mixture of such salts, the stabilizer being capable to form simultaneously water-soluble compounds with hyaluronic acid and/or its pharmaceutically acceptable salt and with decamethoxine and/or its water-soluble salt.

On the assumption that on interaction in solution of non-stabilized decamethoxine and hyaluronic acid a water-insoluble sediment is formed, the stabilizer should be introduced into the mixture before such sediment could possibly form.

So, the subject of the invention is a method for preparing a stabilized solution on base of decamethoxine and hyaluronic acid, wherein HA is added to the pre-formed solution of decamethoxine and stabilizer in water or decamethoxine is added to the pre-formed solution of HA and stabilizer, the stabilizer being selected from the series of pharmaceutically acceptable salts forming only water-soluble compounds rather than water-insoluble precipitate.

Preferably, the method for preparing a stabilized solution on base of decamethoxine and hyaluronic acid consists in decamethoxine being dissolved in solution of stabilizer, after which hyaluronic acid or its salt is dissolved in the solution obtained, or vice versa, stabilizer being dissolved in solution of decamethoxine, after which hyaluronic acid or its salt is dissolved in the solution obtained.

Preferably, the method for preparing a stabilized solution on base of decamethoxine and hyaluronic acid consists in hyaluronic acid or its salt being dissolved in solution of stabilizer, after which decamethoxine is dissolved in the solution obtained, or vice versa, stabilizer being dissolved in solution of hyaluronic acid or its salts, after which decamethoxine is dissolved in the solution obtained.

Preferably, in the method for preparing stabilized solution on base of decamethoxine and hyaluronic acid, pH value of the stabilized solution of decamethoxine and hyaluronic acid is further adjusted to the desired value by adding acid or base.

Preferably, in the method for preparing stabilized solution on base of decamethoxine and hyaluronic acid, said solution is additionally sterilized by autoclaving.

Method for preparing a stabilized solution on base of decamethoxine and hyaluronic acid has been verified in wide range of components ratios, in particular (per 1000 ml of solution):
hyaluronic acid - from 1 g to 50 g,
decamethoxine - from 0.01 g to 10 g,
stabilizer - from 1 g to 60 g.

Decamethoxine may be used in the form of base or chloride.

The stabilized solution on base of decamethoxine and HA may be used to make medicinal preparations by adding supplementary pharmaceutical substances used in medical practice to the stabilized solution to convert it into desired dosage form, from liquid to soft.

Desired dosage forms, in particular, include solution, aerosol, suspension, syrup, drops, solution for injection, application, cream, gel, ointment, paste, liniment, suppository, without being limited thereto.

The stabilized solution also may be itself a pharmaceutical composition.

The stabilized solution and pharmaceutical compositions may be used in any operations having risk of postoperative adhesive process development, in particular:
- abdominal cavity;
- tendon sheaths;
- joints;
- pleural cavity;
- pericardium cavity;
- meninges;
- tympanic cavity;
- coverings of testis
- nerve trunks.

Besides, it may be used in operations having risk of commissure of soft tissues. For example, in neck surgery, where exists a high risk of scar formation throughout all layers of the neck.

The stabilized solution and pharmaceutical compositions on its basis prevent formation of adhesions between organs, and therefore prevent development of adhesive process after operations, relieve inflammation, regenerate injured mucosa, and prevent development of postoperative infections.

With anti-inflammatory purpose, the stabilized solution and pharmaceutical compositions of the present invention may be used to destroy pathogenic flora in gynaecology, stomatology, urology, proctology, ophthalmology, dermatovenereology, the anti-inflammatory action being prolonged, with additional effects of protecting mucous membranes against dehydration, their moistening, and promoting their regeneration.

Other subjects of the invention are pharmaceutical compositions on base of stabilized solution solving other problems stated.

It had been observed that since HA may have broad enough range of molecular weight, it displays different properties. We have differentiated 2 kinds of HA: 1 - HA with molecular weight below 400 kDa, and 2 - HA from 401 kDa to 2,500,000 Da. The first category in the studies described below together with decamethoxine governs penetration of substances to tissue cells, and provides for effective wound-healing action with simultaneous destruction of pathogenic flora, disinfecting action.

Therefore, one more subject solving the above problem, namely, to increase efficiency of treating vaginal infections in order to destroy pathogenic flora in gynaecology, stomatology, urology, proctology, ophthalmology, dermatovenereology, together with providing prolonged anti-inflammatory action to ensure such additional effects as protection of mucous membranes against dehydration, their moistening, promoting their regeneration, decrease in the number of cases of soft tissues traumatism, in particular genital tracts of women in childbirth, decrease in the length of labor and decrease in the incidence of operative delivery, and also in neck surgery, when there exists a high risk of scar formation throughout all layers of the neck; enhanced efficiency of wound healing process with simultaneous bactericidal, antimicrobial action,
is a
pharmaceutical composition on base of stabilized solution of two active substances, characterized in that it contains as first active substance high molecular hyaluronic acid and/or its pharmaceutically acceptable salt (HMHA), the molecular weight of such acid or salts may be from 401 kDa to 2.5 MDa, as second active substance contains decamethoxine and/or its water-soluble salt, stabilizer, which is pharmaceutically acceptable salt capable of dissolving in water and dissotiating in aqueous solutions into metal cations and acid residues anions, or mixture of such salts, the stabilizer being capable to form simultaneously water-soluble compounds with hyaluronic acid and/or its pharmaceutically acceptable salt and with decamethoxine and/or its water-soluble salt, and also contains at least one pharmaceutically acceptable additive.

Preferably, pharmaceutical composition contains substances in the following ratio, mg/ml:

| | |
|---|---|
| hyaluronic acid and/or its pharmaceutically acceptable salt with molecular weight from 401 kDa to 2.5 MDa | 1-50 |
| decamethoxine and/or its water-soluble salt | 0.01-10 |
| stabilizer | 1-60 |
| pharmaceutically acceptable additive | 1-60 |
| water | q.s. to 1 ml |

The technical solution is also a composition containing high-molecular hyaluronic acid with molecular weight from 401 kDa to 2,500,000 Da, decamethoxine, sodium succinate, succinic acid, sodium chloride and water, with following component ratio, mg/ml:

| | |
|---|---|
| hyaluronic acid and/or its pharmaceutically acceptable salt with molecular weight from 401 kDa to 2.5 MDa | 2-8 |
| sodium succinate | 11-22 |
| succinic acid | 0.02-0.08 |
| sodium chloride | 0.8-2 |
| decamethoxine | 0.05-0.5 |
| water | q.s. to 1 ml |

Besides, according to the technical solution, preferably, the stabilized solution contains high-molecular hyaluronic acid with molecular weight from 401 kDa to 2.5 million Da, decamethoxine, sodium succinate, succinic acid, sodium chloride and water, with following component ratio, mg/ml:

| | |
|---|---|
| hyaluronic acid and/or its pharmaceutically acceptable salt with molecular weight from 401 kDa to 2.5 MDa | 5 |
| sodium succinate | 1-60 |
| succinic acid | 0.05 |
| sodium chloride | 1.2 |
| decamethoxine | 0.2 |
| water | q.s. to 1 ml |

Besides, according to the technical solution, preferably, the stabilized solution is used to suppress adhesive process.

Besides, according to the technical solution, preferably, the stabilized solution is used to protect organs against adhesions formation and to avoid organs dehydration.

Also comprises as pharmaceutically acceptable additive at least one useful auxiliary pharmaceutical component to convert it into dosage form from soft to liquid, which may be aerosol, suspension, syrup, drops, solution for injection, cream, gel, ointment, paste, liniment, solution for external use, solution for oral application.

Also comprises as pharmaceutically acceptable additive at least one auxiliary pharmaceutical component from the series of: viscosity regulator, preservative, antioxidant, and comprises as viscosity regulator a substance from the series of alkylcellulose derivative, hydroxyalkyl cellulose derivative, methylcellulose, hydroxypropyl methylcellulose, hydroxybutylcellulose, carboxymethylcellulose, starch, xanthane gum, guar gum, polyacrylic acids and their salts, copolymers of methacrylate, polyethylene oxide, polypropylene oxide, chitin derivatives, starch, pectin, alginic acid, cyclodextrin, agar, and carrageenan; comprises as preservative a substance from the series of lauralkonium chloride, benzalkonium chloride, benzododecinium chloride, cetylpyridinium chloride, cetrimide, domiphen bromide, benzyl alcohol, chlorobutanol, o-cresol, chlorocresol, phenol, ethylphenyl alcohol, benzoic acid, sodium benzoate, sorbic acid, potassium sorbate, paraaminobenzoic acid, methyl parahydroxybenzoate, propyl parahydroxybenzoate, and comprises as antioxidant a substance from the series of: sodium metabisulfite, sodium bisulfite, sodium sulfite, sodium thiosulphate, and ascorbic acid.

Besides, according to the technical solution, preferably, pharmaceutical composition in a dosage form such as gel, comprises as an active component the stabilized solution containing high-molecular hyaluronic acid with molecular weight from 401 kDa to 2.5 million Da, decamethoxine, sodium lactate, lactic acid and water, comprises as an auxiliary pharmaceutical component glycerol and hydroxypropyl methylcellulose, with following component ratio, mg/ml:

| | |
|---|---|
| high-molecular hyaluronic acid with molecular weight from 401 kDa to 2.5 million Da | 2-8 |
| sodium lactate | 0.5-3 |
| lactic acid | 0.1-1 |
| decamethoxine | 0.05-0.5 |
| glycerol | 20-50 |
| hydroxypropyl methylcellulose | 15-50 |
| water | q.s. to 1 ml |

Besides, according to the technical solution, preferably, pharmaceutical composition in a dosage form such as gel, comprises as an active component the stabilized solution containing hyaluronic acid with molecular weight from 401 kDa to 2.5 million Da, decamethoxine, sodium lactate, lactic acid and water, and comprises as an auxiliary pharmaceutical component glycerol and hydroxypropyl methylcellulose, with following component ratio, mg/ml:

| | |
|---|---|
| high-molecular hyaluronic acid with molecular weight from 401 kDa to 2.5 million Da | 5 |
| sodium lactate | 1.42 |
| lactic acid | 0.36 |
| decamethoxine | 0.2 |
| glycerol | 30 |
| hydroxypropyl methylcellulose | 30 |
| water | q.s. to 1 ml |

Besides, according to the technical solution, preferably, pharmaceutical composition is used to suppress adhesive process.

Fourth object of the technical solution according to the present technical solution is a pharmaceutical composition on base of stabilized solution of two active substances, characterized in that the stabilized solution of two active substances comprises as first active substance low-molecular hyaluronic acid and/or its pharmaceutically acceptable salt, the molecular weight of such acid or salts may be from 100 kDa to 400 kDa, decamethoxine and/or its water-soluble salt as second active substance, stabilizer, which is a pharmaceutically acceptable salt capable of dissolving in water and dissotiating in aqueous solutions into metal cations and acid residues anions, or mixture of such salts, the stabilizer being capable to form water-soluble compounds at the same time with hyaluronic acid and/or its pharmaceutically acceptable salt and with decamethoxine and/or its water-soluble salt, and also contains at least one pharmaceutically acceptable additive. Containing components in the following ratio, mg/ml:

| | |
|---|---|
| hyaluronic acid and/or its pharmaceutically acceptable saltwith molecular weight from 100 kDa to 400 kDa | 1-50 |
| decamethoxine and/or its water-soluble salt | 0.01-10 |
| stabilizer | 1-60 |
| pharmaceutically acceptable additive | 1-60 |
| water | q.s. to 1 ml |

Besides, stabilizer is a nontoxic salt or low-toxic salt of inorganic acid, organic monocarboxylic or dicarboxylic acid, or any mixture of such salts, and may be sodium chloride, sodium succinate, sodium lactate, zinc chloride, zinc sulphate, or any mixture of these salts.

A pharmaceutically acceptable salt of low-molecular hyaluronic acid may be sodium hyaluronate.

Besides, comprises as pharmaceutically acceptable additive pH adjustment agent or buffer, in particular phosphate buffer or citrate buffer, or acetate buffer, or succinate buffer, or tris hydrochloride buffer, or maleate buffer, and comprises as pH adjustment agent a substance from the series of: hydrochloric acid, sulfuric acid, nitric acid, phosphoric acid, acetic acid, propionic acid, hydroxyacetic acid, lactic acid, pyruvic acid, oxalic acid, malonic acid, succinic acid, maleic acid, fumaric acid, tartaric acid, citric acid, ascorbic acid, ammonium acetate, ammonia solution, alkaline-earth metal hydroxide, alkali metal hydroxide, sodium carbonate, sodium hydrocarbonate, and sodium phosphate.

One of the embodiments is a pharmaceutical composition comprising sodium succinate as stabilizer, and comprising succinic acid as pH adjustment agent.

One more embodiment is a pharmaceutical composition comprising sodium lactate as stabilizer, and comprising of lactic acid as solution pH adjusting agent.

Such composition may be used to suppress adhesive process, to protect organs against adhesions formation and to heal wounds with simultaneous anti-inflammatory action.

Moreover, may contain as pharmaceutically acceptable additive at least one useful auxiliary pharmaceutical component to convert it into dosage form from soft to liquid, and its dosage form may be aerosol, suspension, syrup, drops, solution for injection, cream, gel, ointment, paste, liniment, solution for external use, and solution for oral application.

The present pharmaceutical composition may contain as pharmaceutically acceptable additive at least one auxiliary pharmaceutical component from the series of viscosity regulator, preservative, and antioxidant, and comprises as viscosity regulator substance from the series of alkylcellulose derivative, hydroxyalkylcellulose derivative, methylcellulose, hydroxypropyl methylcellulose, hydroxybutyl cellulose, carboxymethylcellulose, starch, xanthane gum, guar gum, polyacrylic acids and their salts, copolymers of methacrylate, polyethylene oxide, polypropylene oxide, chitin derivatives, starch, pectin, alginic acid, cyclodextrin, agar, and carrageenan; comprises as preservative a substance from the series of lauralkonium chloride, benzalkonium chloride, benzododecinium chloride, cetylpyridinium chloride, cetrimide, domiphen bromide, benzyl alcohol, chlorobutanol, o-cresol, chlorocresol, phenol, ethylphenyl alcohol, benzoic acid, sodium benzoate, sorbic acid, potassium sorbate, paraaminobenzoic acid, methyl parahydroxybenzoate, and propyl parahydroxybenzoate; comprises as antioxidant a substance from the series of sodium metabisulfite, sodium bisulfite, sodium sulfite, sodium thiosulphate, and ascorbic acid.

One of the embodiments is a pharmaceutical composition, its dosage form is gel containing low-molecular hyaluronic acid and/or its pharmaceutically acceptable salt with molecular weight from 100 kDa to 400 kDa, decamethoxine and/or its water-soluble salt, sodium lactate, lactic acid and water, and containing glycerol and hydroxypropyl methylcellulose as auxiliary pharmaceutical components, at the following components ratio, mg/ml:

| | |
|---|---|
| low-molecular hyaluronic acid and/or its pharmaceutically | 2-8 |
| acceptable salt with molecular weight from 100 kDa to 400 kDa sodium lactate | 0.5-3 |
| lactic acid | 0.1-1 |
| decamethoxine or its water-soluble salt | 0.05-0.5 |
| glycerol | 20-50 |
| hydroxypropyl methylcellulose | 15-50 |
| water | q.s. to 1 ml |

One more embodiment of the composition is a formulation with following composition, mg/ml:

| | |
|---|---|
| low-molecular hyaluronic acid and/or its pharmaceutically | 7 |
| acceptable salt with molecular weight from 100 kDa to 400 kDa sodium lactate | 5 |
| lactic acid | 1.1 |
| decamethoxine or its water-soluble salt | 1 |
| glycerol | 55 |
| hydroxypropyl methylcellulose | 60 |
| water | q.s. to 1 ml |

Also pharmaceutical composition in gel form may have the following composition, mg/ml:

| | |
|---|---|
| low-molecular hyaluronic acid and/or its pharmaceutically | 5 |
| acceptable salt with molecular weight from 100 kDa to 400 kDa sodium lactate | 1.42 |
| lactic acid | 0.36 |
| decamethoxine or its water-soluble salt | 0.2 |
| glycerol | 30 |
| hydroxypropyl methylcellulose | 30 |
| water | q.s. to 1 ml |

Besides, according to the technical solution, preferably, pharmaceutical composition is used to suppress adhesive process.

Besides, according to the technical solution, preferably, pharmaceutical composition is used to accelerate wound healing.

### Description of technical solution

It has been noted above that decamethoxine due to quaternary amino groups has positive charge, while hyaluronic acid due to carboxyl groups is negatively charged, therefore on their interaction in solution a water-insoluble sediment is formed. As a result of studies, it has been found unexpectedly that solution based on two active principles, such as decamethoxine and hyaluronic acid and/or its pharmaceutically acceptable salt, may be obtained by introduction of the stabilizer into the solution. The introduction of stabilizer into the solution containing decamethoxine and hyaluronic acid and/or its pharmaceutically acceptable salt prevents precipitate formation in the solution.

Stabilizer is a pharmaceutically acceptable salt capable of dissolving in water and dissotiating in aqueous solutions into metal cations and acid residues anions, or mixture of said pharmaceutically acceptable salts. Pharmaceutically acceptable salts are nontoxic salts or low-toxic salts. In particular, they may be salts of such acids as inorganic acids (for example, hydrochloric acid, sulfuric acid, and nitric acid), organic mono- and dicarboxylic acids (for example, succinic acid, acetic acid, and lactic acid). The mechanism of stabilization probably lies in the shielding of carboxyl groups of hyaluronic acid and amino groups of decamethoxine in solution by cations and anions of stabilizer, correspondingly.

Effective stabilizers, in particular, are nontoxic salts and low-toxic salts of inorganic acids, salts of organic mono- and dicarboxylic acids, such as, for example, sodium chloride, sodium succinate, sodium lactate, zinc chloride, zinc sulphate. Said salts may be used to obtain, according to the technical solution, the stabilized solution on base of hyaluronic acid and/or its pharmaceutically acceptable salts (hereinafter called for brevity stabilized solution) in any form - both crystalline and in the form of aqueous solution.

According to the technical solution, hyaluronic acid and/or its pharmaceutically acceptable salt may be used in the stabilized solution. Salts such as sodium hyaluronate, potassium hyaluronate, zinc hyaluronate may be used as a salt of hyaluronic acid - according to the technical solution, the preferred pharmaceutically acceptable salt hyaluronic acid is sodium hyaluronate. Salts of hyaluronic acid, similar to hyaluronic acid, are capable to stimulate regenerative processes in tissues, and also display some antimicrobial activity (this is especially so for zinc hyaluronate which has demonstrated certain activity in relation to strains of staphylococcus, streptococcus, and collibacillus). The ability of hyaluronic acid and salts of hyaluronic acid to stimulate regenerative processes in tissues has several different mechanisms of these substances action on the wound healing process - they stimulate phagocytosis, activate granulocytes and macrophages, promote cleansing of wounds from necrotic elements generating local increase in concentration of hyaluronic acid, creates base for cells participating in the wound healing process, induce proliferation of cells such as epithelial cells and fibroblasts, enhance migration of fibroblasts and endothelial cells into injured region of tissues, in its turn create optimal conditions for tissue regeneration processes.

According to the given technical solution, stabilized solution may further contain at least one pharmaceutically acceptable additive. Generally, pH adjustment agent or buffer is used as pharmaceutically acceptable additive. pH adjustment agent or buffer is used to obtain stabilized solution with the required pH value.

pH adjustment agent is a substance or mixture of substances that are added to the stabilized solution in order to obtain the required pH value. As an agent for pH adjustment substances are used from the series of: hydrochloric acid, sulfuric acid, nitric acid, phosphoric acid, acetic acid, propionic acid, hydroxyacetic acid, lactic acid, pyruvic acid, oxalic acid, malonic acid, succinic acid, maleic acid, fumaric acid, tartaric acid, citric acid, ascorbic acid, ammonium acetate, ammonia solution, alkaline-earth metal hydroxide, alkali metal hydroxide, sodium carbonate, sodium hydrocarbonate, and sodium phosphate. The list quoted above puts no limits on substances that can be used according to the present technical solution as pH adjusting agent.

Buffer is a substance or mixture of substances that maintain (stabilize) the pH of the solution at a certain pH value while changing some parameters of the solution (concentration of components, temperature, etc.). Any buffers described in literature and known in the art may be used, in particular, such as phosphate buffer, citrate buffer, acetate buffer, succinate buffer, tris hydrochloride buffer, maleate buffer. The list of the substances quoted puts no limitations on the substances that can be used as buffer according to the present technical solution.

According to the given technical solution, pharmaceutical compositions (preparations) may be prepared on base of stabilized solution by adding auxiliary pharmaceutical components commonly used in medical practice to the stabilized solution, to obtain pharmaceutical compositions in desired dosage form.

Desired dosage forms, in particular, comprise solution, aerosol, suspension, syrup, drops, solution for injection, cream, gel, ointment, paste, liniment, solution for external use, solution for oral application, without being limited thereto.

Dosage forms are easy-to-use states afforded to medicinal preparations (for example, aggregate state, geometrical shape).

By aggregate state, dosage forms are classified into gaseous, liquid, soft and solid.

To the gaseous dosage forms belong gases or gas mixtures, aerosols.

Aerosol is a dosage form that is a disperse system having gaseous dispersion medium and solid or liquid dispersed phase. Aerosols containing liquid dispersed phase are also called sprays. The most important property of aerosols is the ability of solid or liquid particles to remain suspended in dispersion medium after spraying.

To the liquid dosage forms belong solutions, infusions, decoctions, tinctures, extracts, emulsions, suspensions, mixtures, saturations, syrups, and drops.

Solution is a liquid dosage form which is prepared by complete dissolution of solid drug substance in a solvent or mixing of liquids with each other. As a rule, solutions should not contain suspended particles and sediment. Solutions are classified into solutions for external use, solutions for internal use and solutions for injection.

Infusions and decoctions are aqueous extracts of medicinal plants raw materials. They lose their properties rapidly and are therefore prepared immediately prior to the release to patient and in small quantities (for 3-4 days of use).

Tinctures are liquid, transparent, more or less coloured alcoholic, aqueous alcoholic or alcoholic-etheric extracts of drug substances from plant raw materials.

Extracts are concentrated preparations from plant raw materials prepared by using solvents, differ from tinctures in higher concentration of drug substances.

Emulsions are disperse systems consisting of two mutually insoluble liquids, one of which is distributed throughout the other in droplets. Emulsions are prepared by dispersing one liquid within the other, for example, oil in water.

Suspensions are disperse systems consisting of the finest particles of solid substances in liquids. Suspensions are prepared by dispersing solid substances in liquid.

Mixtures are prepared by mixing several drug substances in water, alcohol, and other solvents. They may be transparent, turbid, and even contain sediment, and need to be shaken before their use.

Saturations are liquids saturated with gas.

Syrups are dosage form that is concentrated aqueous solution of sucrose containing drug substances.

Drops is a liquid dosage form intended for internal or external application, and is dosed in drops. As drops, may be used solutions, suspensions, and emulsions.

Soft dosage forms include ointments, pastes, liniments, suppositories, creams, and gels.

Ointments are dosage forms having soft consistency, intended for external use. Ointments are prepared by mixing different medicinal agents with shape-forming substances called ointment bases. As ointment bases, there are used petroleum refining products (petrolatum, paraffin and so on), animal fats and vegetable oils.

Pastes are variety of ointments having powdered substances content not less than 25% (dough-like consistency), therefore they possess good adsorbing and drying properties.

Liniments (also called liquid ointments) are homogeneous mixtures in the form of thick liquids or gelatinous mass melting at body temperature. As liniment base, vegetable oils and animal fats are used.

Suppositories are soft dosage form. They have solid consistency at a room temperature, and melt at body temperature. There are rectal suppositories, vaginal suppositories and sticks. The most suitable base for suppositories is cacao oil.

Creams are soft dosage form for local administration (sometimes they are also called ointments of soft consistency) comprising two- or multiphase disperse systems, often are essentially very thick emulsions of oil-in-water or water-in-oil type. Creams, as opposed to ointments, have less thick consistency, although, similar to ointments, comprise drug substances, oils, fats and other components.

Gels are soft dosage form for local administration, that are single-, two- or multiphase disperse systems with liquid dispersion medium and special disperse phase - gelling agents (substances forming spacial structure in the form of three-dimensional network). Gels are special kind of ointments, which are usually prepared on base of polymer carriers, and due to thick consistency they are able to retain shape, have elasticity and plasticity.

As auxiliary pharmaceutical components to obtain pharmaceutical compositions in desired dosage form, any substances known in the art may be used. In accordance with the present technical solution, generally, pharmaceutical compositions may contain at least one auxiliary pharmaceutical component from the series of viscosity regulator, preservative, and antioxidant. The purpose of these auxiliary pharmaceutical components in pharmaceutical compositions is described in literature and known to those skilled in the art.

As viscosity regulator, may be used a compound from the series of substances, such as: alkylcellulose derivative, hydroxyalkylcellulose derivative, methylcellulose, hydroxypropyl methylcellulose, hydroxybutylcellulose, carboxymethylcellulose, starch, xanthane gum, guar gum, polyacrylic acids and their salts, copolymers of methacrylate, polyethylene oxide, polypropylene oxide, chitin derivatives, starch, pectin, alginic acid, cyclodextrin, agar, and carrageenan. The above list of substances places no limitations on substances that can be used as viscosity regulator according to the present technical solution.

As preservative, may be used a compound from the series of substances, such as lauralkonium chloride, benzalkonium chloride, benzododecinium chloride, cetylpyridinium chloride, cetrimide, domiphen bromide, benzyl alcohol, chlorobutanol, o-cresol, chlorocresol, phenol, ethylphenyl alcohol, benzoic acid, sodium benzoate, sorbic acid, potassium sorbate, PABA, methylparaben, propyl parahydroxybenzoate. The above list of substances places no limitations on substances that can be used as preservative according to the present technical solution.

As antioxidant, may be used a compound from the series of substances, such as sodium metabisulfite, sodium bisulfite, sodium sulfite, sodium thiosulphate, and ascorbic acid. The above list of substances places no limitations on substances that can be used as antioxidant according to the present technical solution.

The stabilized solution itself can also be used as a medicinal agent.

One of the embodiments of the first technical solution is a stabilized aqueous solution with following component ratio, mg/ml:

| | |
|---|---|
| high-molecular hyaluronic acid (HMHA) with molecular weight from 401 kDa to 2.5 mln Da | 5 |
| sodium succinate | 16 |
| succinic acid | 0.05 |
| sodium chloride | 1.2 |
| decamethoxine | 0.2 |
| water | q.s. to 1 ml |

Another embodiment of the third technical solution is a pharmaceutical composition in the form of aqueous gel with following component ratio, mg/ml gel:

| | |
|---|---|
| high-molecular hyaluronic acid (HMHA) with molecular weight from 401 kDa to 2.5 mln Da | 5 |
| sodium lactate | 1.42 |
| lactic acid | 0.36 |
| decamethoxine | 0.2 |
| glycerol | 30 |
| hydroxypropyl methylcellulose | 30 |
| water | q.s. to 1 ml |

One of the embodiments of pharmaceutical composition in the 4^{th} object of technical solution is a stabilized aqueous solution with following component ratio, mg/ml:

| | |
|---|---|
| low-molecular hyaluronic acid (LMHA) with molecular weight from 100 kDa to 400 kDa | 5 |
| sodium succinate | 16 |
| succinic acid | 0.05 |
| sodium chloride | 1.2 |
| decamethoxine | 0.2 |
| water | q.s. to 1 ml |

Another preferred embodiment of the fourth technical solution is a pharmaceutical composition in the form of aqueous gel with following component ratio, mg/ml gel:

| | |
|---|---|
| low-molecular hyaluronic acid (LMHA) with molecular weight from 401 kDa to 2.5 mln Da | 5 |
| sodium lactate | 1.42 |
| lactic acid | 0.36 |
| decamethoxine | 0.2 |
| glycerol | 30 |
| hydroxypropyl methylcellulose | 30 |
| water | q.s. to 1 ml |

The stabilized solution and pharmaceutical compositions on its basis may be used in any operations on organs involving risk of development postoperative adhesive process, in particular, such organs or parts of organs as:
- Abdominal cavity;
- Tendon sheaths;
- Joints;
- Pleural cavity;
- Pericardium cavity;
- Meninges;
- Tympanic cavity;
- Covering of testis
- Nerve trunks;
- Uterine tubes;
- Ovaries;
- Uterus;
- Urinary bladder.

Besides, they may be used in operations on other organs involving risk of commissure of soft tissues. For example, in neck surgery, when there exists a high risk of scar formation throughout all layers of the neck.

The stabilized solution and pharmaceutical compositions on its basis prevent formation of adhesions between organs, and thus prevent development of adhesive process after operations, relieve inflammation, regenerate injured mucosa and prevent development of postoperative infections.

With anti-inflammatory purpose, the stabilized solution and pharmaceutical compositions according to the present technical solution may be used to destroy pathogenic flora in gynaecology, stomatology, urology, proctology, ophthalmology, dermatovenereology, the anti-inflammatory action being prolonged, with additional effect of mucous membranes protection against dehydration, their moistening, and promoting their regeneration.

### Examplary embodiments of the technical solution:

Method for preparing a stabilized solution on base of decamethoxine and high-molecular hyaluronic acid has been tested in wide ranges of component ratios, in particular (per 1000 ml of solution):

| | |
|---|---|
| high-molecular hyaluronic acid or its pharmaceutically acceptable salt | from 1 g to 50 g |
| decamethoxine | from 0.01 g to 10 g |
| stabilizer | from 1 g to 60 g |
| water | q.s. to 1000 ml |

Taking into account that in the course of interaction in solution of decamethoxine and high-molecular hyaluronic acid a water-insoluble sediment is formed, it is obvious that stabilizer should be introduced into the water before one of the components - decamethoxine or hyaluronic acid.

Hyaluronic acid with molecular weight from 401 kDa to 2.5 mln Da due to its large molecular weight is unable to penetrate into cell, however, it had been observed that it envelops organs with a film, thus preventing formation of adhesions and avoiding dehydration of organs. Due to the addition of decamethoxine and/or its salts, simultaneously is achieved also anti-inflammatory and wound healing effects. As a result, wounds are healing faster without adhesive processes and without exacerbation of inflammatory processes, as will be further demonstrated on examples. Such combination of active substances allows to create a film on the surface of organs, which will exhibit simultaneously all the range of the above effects.

Hyaluronic acid with molecular weight from 100 kDa to 400 kDa may penetrate into the cell, therefore its action is more directed towards processes of cells regeneration and wound healing, and in combination with decamethoxine there are yet added bactericidal and anti-inflammatory properties. Even though the molecular weight is not large, such low-molecular hyaluronic acid also is able to form a film over organs, nevertheless with less effect on the adhesive process, as it will be demonstrated in the examples below, providing at that different range of action.

### Specific embodiments

Hereinafter are described the examples of specific embodiments of the technical solution.

### Example 1.

### Preparation of stabilized solution and pharmaceutical composition on its basis

800 ml of purified distilled water are placed into a flask. 16 g of sodium succinate, 50 mg of succinic acid, 1.2 g of sodium chloride are added sequentially into the flask and mixed for 5-10 min on magnetic stirrer (600 rpm) until complete dissolution of the components. After that, 200 mg of decamethoxine is added to the solution under continuous mixing until complete dissolution. 5 g of high-molecular hyaluronic acid (from 401 kDa to 2.5 million Da) is added and mixed on magnetic stirrer to complete hydratation (dissolution) of high-molecular hyaluronic acid.

Thereafter, pH of the stabilized solution obtained, if needed, is adjusted to 7.3 with succinic acid, after which volume of the solution prepared is brought to 1000 ml with purified water.

### Example 2.

### Preparation of stabilized solution.

The procedure of Example 1 is repeated, except for sodium hyaluronate being used instead of high-molecular hyaluronic acid.

### Example 3.

### Preparation of pharmaceutical composition on base of stabilized solution in gel form.

800 ml of purified distilled water is poured into the flask. 200 mg of decamethoxine, 1.42 g of sodium lactate, 360 mg of lactic acid, 30 g of glycerol are added sequentially into the flask and mixed for 5-10 min on magnetic stirrer (600 rpm) until complete dissolution of the components. After that, 5 g high-molecular hyaluronic acid (from 401 kDa to 2.5 million Da) is added to the solution under continuous mixing and mixed on magnetic stirrer to complete hydratation (dissolution) of hyaluronic acid. 30 g of hydroxypropyl methylcellulose is added to the solution, after which volume of the mixture is brought to 1000 ml with purified water with mixing. If needed, pH of the solution is adjusted to 7.315 by adding lactic acid or sodium lactate.

Method for preparing a stabilized solution on base of decamethoxine and low-molecular hyaluronic acid has been tested in a wide range of component ratios, in particular (per 1000 ml of solution):

| | |
|---|---|
| low-molecular hyaluronic acid and/or its pharmaceutically acceptable salt | from 1 g to 50 g |
| decamethoxine | from 0.01 g to 10 g |
| stabilizer | from 1 g to 60 g |
| water | q.s. to 1000 ml |

On the assumption that in the course of interaction of decamethoxine and low-molecular hyaluronic acid in solution, a water-insoluble sediment is formed, the stabilizer obviously should be addet to water before one of the components - decamethoxine or hyaluronic acid.

### Example 4.

### Preparation of pharmaceutical composition on base of stabilized solution

800 ml of purified distilled water is poured into a flask. 16 g of sodium succinate, 50 mg of succinic acid, and 1.2 g of sodium chloride are Added sequentially into the flask: and mixed for 5-10 min on magnetic stirrer (600 rpm) until complete dissolution of the components. After that, 200 mg of decamethoxine is added to the solution under continuous mixing until complete dissolution. 5 g of low-molecular hyaluronic acid (from 100 kDa to 400 kDa) is added and mixed on magnetic stirrer to complete hydratation (dissolution) of hyaluronic acid. If needed, pH of the solution is adjusted to 7.3 with succinic acid, after which volume of the solution prepared is brought to 1000 ml with purified water.

### Example 5.

### Preparation of pharmaceutical composition on base of stabilized solution.

The procedure of Example 4 is repeated, except for sodium hyaluronate being used instead of low-molecular hyaluronic acid.

### Example 6.

### Preparation of pharmaceutical composition on base of stabilized solution in gel form.

800 ml of purified distilled water is poured into flask. 200 mg of decamethoxine, 1.42 g of sodium lactate, 360 mg of lactic acid, and 30 g of glycerol are added sequentially into the flask and mixed for 5-10 min on magnetic stirrer (600 rpm) until complete dissolution of the components. After that, 5 g of low-molecular hyaluronic acid (from 100 kDa to 400 kDa) is added under continuous mixing to the solution and mixed on magnetic stirrer to complete hydratation (dissolution) of hyaluronic acid. 30 g of hydroxypropyl methylcellulose is added to the solution, after which volume of the mixture is brought to 1000 ml with purified water under mixing. If needed, pH of the solution is adjusted to 7.315 by adding lactic acid or sodium lactate.

### Example 7. Preparation of stabilized solution.

800 ml of purified distilled water is poured into flask, 200 mg of decamethoxine and 1.42 g of sodium lactate are added at continuous mixing for 5-10 minutes on magnetic stirrer (600 rpm) until complete dissolution of the components. Thereafter, 5 g of low-molecular hyaluronic acid (from 100 kDa to 400 kDa) is added at continuous mixing and mixed on magnetic stirrer to complete hydratation (dissolution) of hyaluronic acid.

In order to prepare pharmaceutical composition 360 mg of lactic acid and 30 g of glycerol are added sequentially and mixed 5-10 min on magnetic stirrer (600 rpm) until complete dissolution of the components. 30 g of hydroxypropyl methylcellulose is added to the solution, after which volume of the mixture is brought to 1000 ml with purified water under mixing.

To determine properties of stabilized solutions and pharmaceutical compositions prepared according to the technical solution, such as stability of stabilized solutions, antibacterial activity of stabilized solutions and pharmaceutical compositions on their basis, studies have been performed of the activity of pharmaceutical compositions on the adhesions development, effects of pharmaceutical compositions on wounds healing, with results being described below.

In the course of the studies, the stabilized solutions prepared in Examples 2 and 4 were stored at constant room temperature for 2 years, and at certain time intervals were checked for presence/absence of sediment in the stabilized solutions, sodium hyaluronate content in stabilized solutions, and pH value of stabilized solutions. The results of the studies are presented in Table 1

**Table 1**

| Storage temperature (°C) | Storage life (months) | Presence/absence of sediment | Sodium hyaluronate content (HMHA) mg/ml | pH value (HMHA) | Sodium hyaluronate content (LMHA) mg/ml | pH value (LMHA) |
|---|---|---|---|---|---|---|
| 25 | 0 | Transparent solution, no precipitate | 5.1 | 7.31 | 5.1 | 7.31 |
| 25 | 3 | Transparent solution, no precipitate | 5.1 | 7.31 | 5.1 | 7.31 |
| 25 | 6 | Transparent solution, no precipitate | 5.1 | 7.31 | 5.1 | 7.31 |
| 25 | 9 | Transparent solution, no precipitate | 5.05 | 7.3 | 5.05 | 7.3 |
| 25 | 12 | Transparent solution, no precipitate | 5.05 | 7.3 | 5.05 | 7.3 |
| 25 | 15 | Transparent solution, no precipitate | 5.05 | 7.31 | 5.05 | 7.31 |
| 25 | 15 | Transparent solution, no precipitate | 5.05 | 7.31 | 5.05 | 7.31 |
| 25 | 18 | Transparent solution, no precipitate | 5.1 | 7.29 | 5.1 | 7.29 |
| 25 | 21 | Transparent solution, no precipitate | 5.0 | 7.29 | 5.0 | 7.29 |
| 25 | 24 | Transparent solution, no precipitate | 5.0 | 7.29 | 5.0 | 7.29 |

As it is seen from data in Table 1, the stabilized solutions prepared doesn't change their properties during two years storage at room temperature.

Studies of antibacterial activity of the stabilized solutions prepared in Examples 2 and 4 were conducted using standard method of by paper discs. The results of the studies are presented in Table 2.

**Table 2**

| Temper ature of the studies (°C) | Thermo stating time, hours | Paper disc exposure time in solution, minutes | Decameth oxine content, mg/ml (HMHA solution) | Growth delay zone of Staphylococcus aureus, mm (HMHA solution) | Decameth oxine content, mg/ml (LMHA solution) | Growth delay zone of Staphylococcus aureus, mm (LMHA solution) |
|---|---|---|---|---|---|---|
| 37 | 24 | 2 | 0.2 | 1.2 | 0.2 | 1.2 |
| 37 | 48 | 2 | 0.2 | 1.4 | 0.2 | 1.4 |

There have been conducted studies to examine effects of pharmaceutical compositions according to the technical solution (compositions of pharmaceutical preparations indicated in Examples 2 and 4) on the development of adhesions in abdominal cavity of rats under conditions of abdominal surgical intervention and inflicting mechanical injury (mesentery stitching with ligature and scarification of serous layer of intestine) and chemical injury (application of powdered Altabor tablets and application of iodine solution on serous layer of intestine to activate the adhesive process.

In the experiment have been used 66 white unbred rats with mass of 180-200 g, which were randomized into experimental groups designated as follows:
- Control pathology 1;
- Ligature + Pharmaceutical composition (HMHA, LMHA);
- Control pathology 2;
- Scarification + Pharmaceutical composition (HMHA, LMHA);
- Control pathology 3;
- Powdered Altabor tablets + Pharmaceutical composition (HMHA, LMHA);
- Control pathology 4;
- Iodine solution + Pharmaceutical composition (HMHA, LMHA).

In four experimental animals groups, injuring factor was a mechanical one - in two experimental groups the injury was inflicted with ligature (stitching of duodenal mesentery with silk thread), and in two experimental animals groups the injury was inflicted by scarification (drawing 10 longitudinal scratches with point of a surgical needle until "bloody dew" on the serous layer of cecal appendage).

In four experimental animals groups injuring factor was a chemical one - in two experimental groups the injury was inflicted by application of powdered Altabor tablets (30 mg) to the visceral surface of spleen, and in two experimental groups the injury was inflicted by application of alcoholic iodine solution onto the cecal appendage.

Table 3 lists designations of experimental animals groups, factors influencing the process of adhesions formation in each experimental group, injuring factor, and number of animals in each experimental group.

**Table 3**

| Designation of experimental group (in parentheses factors influencing the process of adhesions formation) | Injuring factor | Number of rats in experimental group |
|---|---|---|
| Mechanical injuring factor | | |
| Control pathology 1 (injuring factor only - ligature) | Duodenal mesentery stitching with silk thread | 6 |
| Ligature + Pharmaceutical composition (injuring factor - ligature, and administration of pharmaceutical composition of HMHA and LMHA) | | 12 |
| Control pathology 2 (injuring factor only - scarification) | Scarification of serous layer of cecal appendage (10 longitudinal scratches with point of a surgical needle until "bloody dew") | 6 |
| Scarification + Pharmaceutical composition (injuring factor - scarification, and administration of pharmaceutical composition of HMHA and LMHA) | | 12 |

| Chemical injuring factor | | |
|---|---|---|
| Control pathology 3 (injuring factor only - powdered Altabor tablets) | Application of powdered Altabor tablets (30 mg) on the visceral surface of spleen | 6 |
| Powdered Altabor tablets + Pharmaceutical composition (injuring factor - powdered Altabor tablets, and administration of pharmaceutical composition of HMHA and LMHA) | | 12 |
| Control pathology 4 (injuring factor only - iodine solution) | Application of alcoholic iodine solution onto the cecal appendage | 6 |
| Iodine solution + Pharmaceutical composition (injuring factor - iodine solution, and administration of pharmaceutical composition of HMHA and LMHA) | | 12 |

After randomization, animals were subjected to abdominal surgical intervention under anesthesia with sodium thiopental introduced intraabdominally in dose of 50 mg/kg. After midline laparotomy, injuries of abdominal cavity organs have been inflicted to animals in experimental groups by one of the following methods, in accordance with randomization:
1) stitching of a portion of duodenal mesentery with silk thread;
2) scarification of serous tunic of cecal appendage (10 longitudinal scratches with point of a surgical needle until "bloody dew");
3) dusting the visceral surface of spleen with powdered Altabor tablets (30 mg);
4) application of 5% alcoholic iodine solution to serous tunic of cecal appendage.

After injuring corresponding organs of animals in the experimental groups, pharmaceutical compositions were introduced into abdominal cavity in the amount of 5 ml. After that, abdominal cavity of experimental animals was sutured. Animals were observed for 14 days. State of the animals was evaluated by determination of survival rate, assessment of appearance, and body weight dynamics. On Day 14 animals were removed from the experiment by anaesthetic overdose with abdominal cavity opened, macroscopic review performed, presence of adhesions determined, and state of the organs and tissues in the locations of manipulations scored. Thereafter, fragments of the complex of matted organs were dissected within the limits of adhesions, weighted and fixed in 10% formalin solution in order to conduct morphological studies. Then samples of adhesive tissues were dehydrated in alcohols and embedded in celloidin-paraffin. Sections were stained with hematoxylin and eosin, picrofuchsin according to Van Gieson. Microscopic studies of micropreparations were conducted using microscope Granum. Photomicrography of images was performed using digital video camera Granum DSM 310. Photographs were computer processed using Toup View program.

To score the state of the organs and tissues in the locations of manipulations, following scale was used:

### Portion of duodenal mesentery:

0 - no changes;
0.5 - whitish infiltrations at the threads;
1 - whitish infiltrations at the threads + punctate filamentary (in the form of bundles) adhesion with adjacent loop of large intestine;
2 - whitish infiltrations at the threads + planar (broad collision) adhesions with adjacent loop of large intestine;
3 - whitish infiltrations at the threads + planar adhesions with adjacent loop of large intestine + adhesions with the caudate lobe of the liver and/or with pancreas near the gastrolienal ligament.

### Serous tunic of cecal appendage:

0 - no changes;
1 - small whitish thickening on the surface of tip of the appendix;
2 - moderate whitish thickening on the surface of tip of the appendix + punctate filamentary adhesions between appendix body and adjacent loop of small intestine;
3 - marked whitish thickening on the surface of the appendix + planar adhesions between appendix body and adjacent loop of small intestine.

### Spleen capsule

0 - no changes;
1 - small isolated whitish thickenings on the diaphragmatic surface of organ;
2 - whitish thickenings spreading on the diaphragmatic surface of organ;
3 - whitish thickenings spreading on the diaphragmatic surface of organ;
4 - whitish thickening spreading on the diaphragmatic surface of organ and outside + adhesions of visceral surface of the organ (its gastric region) with adjacent caudal part of pancreas.

Statistical treatment has been conducted using parametric and non-parametric methods of single factor analysis of variance - Kruskal-Wallis and Mann-Whitney tests, and Fisher angular transformation.

Studies of the pharmaceutical compositions effects on the development of adhesions in abdominal cavity of rats after mechanical injury of a portion of duodenal mesentery caused by stitching with silk thread have demonstrated following results.

After mechanical injury of a portion of duodenal mesentery caused by stitching with silk thread, no significant impairments of trophic processes and general body state have been observed in animals from control and test groups, as indicated by dynamics of the body weight of rats, preesented in Table 4.

**Table 4**

| Time of experiment | Rats' body weight, g | | |
|---|---|---|---|
| | Experimental group - Control pathology 1 | Experimental group - Ligature + Pharmaceutical composition of HMHA | Experimental group - Ligature + Pharmaceutical composition of LMHA |
| Initial state | 194.17±12.38 | 185.00±10.82 | 194.17±12.38 |
| 3 days | 190.00±10.29 | 180.83±10.53 | 197.00±10.29 |
| 7 days | 201.67±10.67 | 186.67±10.67 | 201.67±10.67 |
| 14 days | 205.83±10.83 | 177.50±12.81 | 205.83±10.83 |

As a result of macroscopic studies of adhesive process development in abdominal cavity of rats it has been established that after mechanical injury of a portion of duodenal mesentery caused by stitching with silk thread in animals from the group of Control pathology 1 development of the adhesive process had been observed in 100% animals (6 of the 6). In experimental group of Ligature + Pharmaceutical composition, the pharmaceutical composition of HMHA promotes reliable decrease in the number of animals with adhesions by 83,3% (1 out of 6) in comparison with the group of Control pathology 1, and pharmaceutical composition of LMHA - by 50%, as it is seen from data in Table 5.

The data presented indicate that pharmaceutical composition of LMHA also promotes decrease in adhesions formation, although not as convincingly as pharmaceutical composition containing HMHA. However, effect of the pharmaceutical composition of HMHA on the adhesions formation is quite obvious.

**Table 5**

| Experimental group | Number of animals in experimental group | Number of animals with adhesions | Fraction of animals with adhesions, % |
|---|---|---|---|
| Control pathology 1 | 6 | 6 | 100 |
| Ligature + Pharmaceutical composition of HMHA | 6 | 1 | 16.7* |
| Ligature + Pharmaceutical composition of LMHA | 6 | 3 | 50 |

| | | | |
|---|---|---|---|
| Note: * - value deviation, estimated using Fisher angular transformation, value significant relative to the group of Control pathology 1, p <0.05. | | | |

Score estimation of pronouncedness of adhesive process presented in Table 6 demonstrates significant antiadhesive activity of pharmaceutical compositions of HMHA and LMHA, as indicated by significant decrease in the score values of adhesive process by 4.2-fold for pharmaceutical composition of HMHA, and by 1.8-fold for pharmaceutical composition of LMHA, in relation to the group of Control pathology 1.

**Table 6**

| Experimental group | Number of animals in experimental group | Pronouncedness of adhesive process, score |
|---|---|---|
| Control pathology 1 | 6 | 2.33±0.21 |
| Ligature + Pharmaceutical composition of HMHA | 6 | 0.56±0.06* |
| Ligature + Pharmaceutical composition of LMHA | 6 | 1,3±0.04 |

| | | |
|---|---|---|
| Note: * - value deviation, estimated by Mann-Whitney method, value significant relative to the group of Control pathology 1, p <0.05 | | |

Determination of mass of the complex of matted organs in rats after stitching of duodenal mesentery with silk threads (Table 7) also demonstrates that pharmaceutical compositions promote significant decrease of mass of the complex of matted organs by 1.6-fold for pharmaceutical composition of HMHA and by 1.2-fold for pharmaceutical composition of LMHA in comparison with Control pathology 1 group, also indicating to the pronounced antiadhesive action of the pharmaceutical composition of HMHA.

**Table 7**

| Experimental group | Mass of the complex of matted organs, g |
|---|---|
| Control pathology 1 | 1.04±0.10 |
| Ligature + Pharmaceutical composition of HMHA | 0.67±0.05* |
| Ligature + Pharmaceutical composition of LMHA | 0.87±0.05 |

| | |
|---|---|
| Note: * - value deviation estimated by Newman-Keuls method, value significant relative to the group of Control pathology 1, p <0.05 | |

Studies of macropreparations of the complex of matted organs in the location of duodenal mesentery stitching demonstrates that in the group of Control pathology 1 66.7% of rats have well-seen whitish infiltrations at the threads and planar adhesions with adjacent loop of large intestine, while 33.3% of animals have also adhesions with the caudate lobe of the liver.

In the group of Ligature + Pharmaceutical composition administered the pharmaceutical composition of HMHA, minimal changes in the location of the stitching were observed in 83.3% of animals, characterized by whitish thickenings at the threads. Only one rat (16,7% or 1 out of 6) these thickenings were combined with single punctate filamentary adhesions with adjacent loop of large intestine.

In animals from the Control pathology 1 group, in the region of stitching and ripple marks around remnants of threads there are formed different in size foci of fibrous tissue wherein giant cells of foreign-body type (macrophages) are observed. As a rule, no inflammatory reaction is manifest in adjacent locations of the mesentery. In the same zone a whole adhesive complex if often formed comprising adherent loops of small and large intestine, and in some cases also lobe of liver, and small adhesive regions of liver and pancreas. The adhesions were characterized as already clearly oriented, significantly fuchsinophilic collagen fiber bundles, and also more loose structure of a granular type with collagen fibrillae having vaguely longitudinal orientation. In the tissue of adhesions giant cells of foreign-body type are seen here and there in immediate vicinity to serous membrane of intestine walls. In muscular tunic of intestine pathological changes are detected in the form of dystrophy and disorganization of muscular fibrils, penetration of collagen fibers into muscular tissue. In 66.7% of animals fibrin masses are deposited in locations of trauma, filamentary and planar adhesions develop between omentum and adjacent loop of large intestine (2 points). In 33.3% of animals adhesions with capsule of the caudate lobe of liver are also noted (3 points).

In animals from the experimental group Ligature + Pharmaceutical composition of HMHA, in the zone of intervention, there were determined substantially smaller in size in comparison with the experimental group Control pathology 1, incapsulated granulomas of foreign bodies, non-resolved fragments of threads, with small petches of fibrous tissue around, were surrounded with sufficiently thin mature connective-tissue capsule. No adhesions of intestine loops in the region of stitching were found in overwhelming majority of rats, with only "coarsening" of layers of loose fibrous connective tissue separating lobes of adipose tissue of omentum noted in some locations. Small adhesion between intestinal loops had been found in one rat only.

Studies of the effects of pharmaceutical compositions on the development of adhesions in abdominal cavity of rats after mechanical injury of serous membrane of cecal appendage inflicted by scarification gave following results.

No significant disturbances of trophic processes and body's general functional status have been observed after mechanical injury of serous membrane of cecal appendage inflicted by scarification in animals from the experimental groups, as indicated by dynamics of rats' body weight - it is seen from data presented in Table 8.

**Table 8**

| | Rats' body weight, g | | |
|---|---|---|---|
| Time of experiment | Experimental group - Control pathology 2 | Experimental group - Scarification + Pharmaceutical composition of HMHA | Experimental group - Scarification + Pharmaceutical composition of LMHA |
| Initial state | 196.67±5.47 | 185.00±6.83 | 185.00±6.83 |
| 3 days | 187.50±6.09 | 180.83±1.54 | 180.83±1.54 |
| 7 days | 201.67±3.80 | 186.67±10.67 | 186.67±10.67 |
| 14 days | 205.00±4.47 | 187.40±11.72 | 187.40±11.72 |

It has been established as a result of macroscopic studies of adhesive process development in abdominal cavity of rats that after mechanical injury of serous membrane of cecal appendage inflicted by scarification, development of the adhesive process in animals from the group of Control pathology 2 had been observed in 100% of cases (6 out of 6). In experimental group Scarification + Pharmaceutical composition, pharmaceutical composition of HMHA promotes reliable decrease, in comparison with the group of Control pathology 2, in the number of animals with adhesions down to 16.7% (1 out of 6), and pharmaceutical composition of LMHA - by 50% (3 out of 6), as it is seen from data in Table 9.

**Table 9**

| Experimental group | Number of animals in experimental group | Number of animals with adhesions | Percentage of animals with adhesions, % |
|---|---|---|---|
| Control pathology 2 | 6 | 6 | 100 |
| Scarification + Pharmaceutical composition of HMHA | 6 | 1 | 16.7* |
| Scarification + Pharmaceutical composition of LMHA | 6 | 3 | 50 |

| | | | |
|---|---|---|---|
| Note: * - value deviation estimated using Fisher angular transformation, value significant relative to the group of Control pathology 2, p <0.05. | | | |

Score estimation of pronouncedness of adhesive process presented in Table 10 testifies to the substantial antiadhesive effect of pharmaceutical composition of HMHA, as indicated by significant, 3.2-fold, decrease in score points in comparison with the group of Control pathology 2, and antiadhesive action of pharmaceutical composition of LMHA, by 1.67-fold in comparison with control group 2.

**Table 10**

| Experimental group | Number of animals in experimental group | Pronouncedness of adhesive process, score |
|---|---|---|
| Control pathology 2 | 6 | 2.00±0,00 |
| Scarification + Pharmaceutical composition of HMHA | 6 | 0,63+0.26* |
| Scarification + Pharmaceutical composition of LMHA | 6 | 1.2±0.00 |

| | | |
|---|---|---|
| Note: * - value deviation estimated by Mann-Whitney method, value significant relative to the group of Control pathology 2, p <0.05. | | |

Determination of mass of the dissected complex of matted organs in rats after mechanical injury of a portion of serous membrane of cecal appendage inflicted by scarification (see Table 11) also demonstrates that pharmaceutical composition of HMHA promotes decrease of mass of the complex of matted organs by 1.5-fold, reliable in comparison with the group of Control pathology 2, and pharmaceutical composition of LMHA - by 1.2-fold, also indicative of pronounced antiadhesive action of the pharmaceutical composition of HMHA.

**Table 11**

| Experimental group | Mass of the complex of matted organs, g |
|---|---|
| Control pathology 2 | 0,99±0.08 |
| Scarification + Pharmaceutical composition of HMHA | 0.67±0.05* |
| Scarification + Pharmaceutical composition of LMHA | 0.8±0.05 |

| | |
|---|---|
| Note: * - value deviation estimated by Newman-Keuls method, value significant relative to the group of Control pathology 2, p <0.05. | |

Studies of macropreparations of the complex of matted organs in the location of injury of a portion of serous membrane of cecal appendage inflicted by scarification demonstrate that in 100% of rats (6 out of 6) in the group of Control pathology 2 there were found moderate whitish thickening on the surface of tip of the appendix and isolated punctate filamentary adhesions between appendix body and overlying loop of small intestine.

In the group of Scarification + Pharmaceutical composition of HMHA, no visible changes of tissues were found in 50% of rats in the location of intervention. In 33.3% of animals small whitish thickenings were observed on the surface of tip of the appendix, and in one animal (16.7%), additionally, also isolated punctate filamentary adhesion with adjacent loop of small intestine.

Morphological studies of adhesive process development in abdominal cavity of rats after mechanical injury of a portion of serous membrane of cecal appendage inflicted by scarification demonstrate that in the group of Control pathology 2 on the surface of serous membrane of tip of appendix there are observed moderately large fibrin masses, destroying it by not only close contacting with muscular tunic, but also partly replacing muscular fibrils. Fibrin accumulations have in general loose filamentary, fuchsinophilous character, variable filling with cells. In the region of appendix body, obviously, traces of trauma and inflammation affecting submucous layer and muscular tunic are retained in area of immediate mechanical impact. In some locations, omentum of unaltered appearance is located close to serous tunic of appendix body, in some places it "penetrates" into muscular fibrils, and in separate locations it is transformed into adhesion of mature appearance extending also to the adjacent loop of small intestine. Therefore, less pronounced adhesive process develops after scarification of the surface of cecal appendage - 2 score points In all rats, in addition to deposits of fibrin masses, punctate filamentary adhesions with corresponding loop of small intestine were found on the surface of the appendix.

In the group of Scarification + Pharmaceutical composition of HMHA, fibrin thickening on the surface of tip of cecal appendage were absent in 50% of rats. Omentum without changes, adjoins loosely to the body of appendix. In 33.3% of animals, moderate fibrin thickenings were observed on serous tunic of tip of the appendix. Connective-tissue layers of omentum were roughened in places, but no formation of adhesions with loop of small intestine was observed. and isolated punctate adhesions with loop of small intestine were found in only 16.7% (in 1 out of 6) of rats. Collagen fibers of adhesions are less mature than in animals from the group of Control pathology 2, no integrity violations of muscular tunic of intestine were observed.

Thus, intraabdominal administration of pharmaceutical composition of HMHA after scarification of the surface of cecal appendage prevents development of adhesions in 50% of animals, and decreases process of adhesions development in 33.3% of rats. Pharmaceutical composition promotes morphologically decrease in the volume of fibrin masses deposited in the locations of intervention, and also decreases volume and spreading of adhesions, prevents accelerated maturation of adhesions' tissue.

Studies of pharmaceutical compositions effects on the development of adhesions in abdominal cavity of rats after injury of visceral surface and capsule of spleen caused by powdered Altabor tablets, gave following results.

No significant disturbances of trophic processes and body's general functional status have been observed after chemical injury of visceral surface and capsule of spleen caused by powdered Altabor tablets in animals from experimental groups, as indicated by dynamics of rats' body weight - see data presented in Table 12.

**Table 12**

| | Rats' body weight, g | | |
|---|---|---|---|
| Time of experiment | Experimental group - control pathology 3 | Experimental group - powdered Altabor tablets + Stabilized solution HMHA | Experimental group - powdered Altabor tablets + Stabilized solution LMHA |
| Initial state | 231.66±3.33 | 198.33±3.07 | 200.33±3.07 |
| 3 days | 225.83±4.17 | 204.17±4.16 | 204.4±4.16 |
| 7 days | 225.83±5.07 | 205.83±3.27 | 205.83±3.27 |
| 14 days | 225.83±5.07 | 196.67±3.57 | 205.67±2.57 |

As a result of macroscopic studies of adhesive process development in abdominal cavity of rats, it has been established that development of the adhesive process had been observed in 100% of animals (6 out of 6) after injury of visceral surface and capsule of spleen caused by powdered Altabor tablets, in animals from the group of Control pathology 3. In the experimental group of powdered Altabor tablets + Pharmaceutical composition, pharmaceutical composition of HMHA promotes reliable decrease by 83.3% (1 out of 6) in the number of animals with adhesions in comparison with the group of Control pathology 3, and pharmaceutical composition of LMHA - by 50%, as it is seen from data in Table 13.

**Table 13**

| Experimental group | Number of animals in experimental group | Number of animals with adhesions | Percentage of animals with adhesions, % |
|---|---|---|---|
| Control pathology 3 | 6 | 6 | 100 |
| Powdered Altabor tablets + Pharmaceutical composition of HMHA | 6 | 1 | 16.7* |
| Powdered Altabor tablets + Pharmaceutical composition of LMHA | 6 | 3 | 50 |

| | | | |
|---|---|---|---|
| Note: * - value deviation estimated using Fisher angular transformation, value significant relative to the group of Control pathology 3, p <0.05. | | | |

Score estimation of pronouncedness of adhesive process presented in Table 14 testifies to substantial antiadhesive activity of pharmaceutical composition of HMHA, as indicated by significant decrease, in relation to the group of Control pathology 3, of score points by 2.3-fold, and antiadhesive activity of pharmaceutical composition of LMHA - by 1.5-fold.

**Table 14**

| Experimental group | Number of animals in experimental group | Pronouncedness of adhesive process, score |
|---|---|---|
| Control pathology 3 | 6 | 4.00±0,00 |
| Powdered Altabor tablets + Pharmaceutical composition of HMHA | 6 | 1.71±0,64* |
| Powdered Altabor tablets + Pharmaceutical composition of LMHA | 6 | 2.65±0.5 |

| | | |
|---|---|---|
| Note: * - value deviation estimated by Mann-Whitney method, value significant relative to the group of Control pathology 3, p <0.05 | | |

Determination of mass of the dissected complex of matted organs in rats after chemical injury of visceral surface and capsule of spleen caused by powdered Altabor tablets (see Table 15) also demonstrates that pharmaceutical composition of HMHA promotes decrease of mass of the complex of matted organs by 1.7-fold, reliable in comparison with the group of Control pathology 3, and pharmaceutical composition of LMHA by 1.26-fold, which also indicates to the more pronounced antiadhesive action of pharmaceutical composition of HMHA.

**Table 15**

| Experimental group | Mass of the complex of matted organs, g |
|---|---|
| Control pathology 3 | 1.77±0.10 |
| Powdered Altabor tablets + Pharmaceutical composition of HMHA | 1 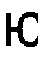 05±0,06* |
| Powdered Altabor tablets + Pharmaceutical composition of LMHA | 1.4±0.05 |

| | |
|---|---|
| Note: * - value deviation estimated by Newman-Keuls method, value significant relative to the group of Control pathology 3, p <0.05 | |

Studies of macropreparations of the complex of matted organs in the location of injury of visceral surface and capsule of spleen caused by powdered Altabor tablets demonstrate that in the group of Control pathology 3 in 100% of rats (6 out of 6) changes were characterized by whitish depositions on the diaphragmatic surface spreading over large part of the organ's surface. These changes were combined with planar adhesions between visceral surface of spleen (its gastric region) and adjacent caudal part of pancreas.

There were no visible changes on the surface of organ in the group of Powdered Altabor tablets + Pharmaceutical composition of HMHA in 33.3% of rats. The whitish thickenings were spreading over the diaphragmatic surface of organ in 16.7% of rats, and over the visceral surface of organ in 33.3% of rats. Only in 16.7% of rats whitish thickening spread over the diaphragmatic surface of spleen were combined with small adhesions between gastric region of the visceral surface and adjacent caudal part of pancreas.

Morphological studies of adhesive process development in abdominal cavity of rats after injury of visceral surface and capsule of spleen caused by powdered Altabor tablets, demonstrate that thickness of capsule of the diaphragmatic surface of organ in rats from the group of Control pathology 3 is sharply increased due to proliferation of collagen fibers. In some places there are observed also thickenings of trabecules given off the capsule. In a number of cases, sufficiently large foci of inflammation were observed in thickened capsule. In all animals there were observed planar adhesions between capsule of the visceral surface of spleen and cellular tissue surrounding pancreas. In these adhesions base of tissue was represented by a vast network of newly oriented collagen fibers. In some places among fibers are observed separate giant cells of foreign-body type. Picrofuchsin staining according to Van Gieson demonstrate that collagen fibers both in capsule and in adhesions are sufficiently mature, and expressly fuchsinophilic. In a number of animals were found commissures that are also formed - adhesion of regions of omentum with cellular tissue surrounding lobes of pancreas. They are sufficiently large fields of dense cell tissue with inclusions of randomly arranged collagen fibrils. In parenchyma of the gland, inflammatory phenomena were observed. Thus it is seen that after application of foreign body - powdered Altabor tablets - on visceral surface of spleen, severe adhesive process is developing. In 100% of cases, deposition of dense fibrin masses takes place, which coalesce with capsule of the diaphragmatic surface, and development of dense planar adhesions between portions of omentum and visceral surface of spleen, and also between cellular tissue of pancreas and capsule of the caudate lobe of liver - 4 score points

In the group of Powdered Altabor tablets + Pharmaceutical composition of HMHA in 33.3% of rats capsule of the diaphragmatic surface of spleen remains without changes or changes were very insignificant - somewhere, fibrin fibers are observed on the surface of somewhat thickened capsule, isolated giant cells of foreign-body type. Visceral surface of organ remains intact. In other animals moderate variable thickening of capsule of the diaphragmatic surface is noted. Collagen fibers are somewhat less densely packed therein in comparison with rats from the group of Control pathology 3. On the visceral surface there are observed adhesions with cellular tissue surrounding lobes of pancreas, but opposite to control, base of adhesions is represented by less mature delicate loose filamentary tissue.

Studies of pharmaceutical compositions effects on the development of adhesions in abdominal cavity of rats after chemical injury of cecal appendage caused by alcoholic iodine solution, gave following results.

After chemical injury of cecal appendage caused by 5% alcoholic iodine solution, no significant disturbances have been observed in trophic processes and body's general functional status in animals from experimental groups, as indicated by dynamics of rats' body weight - see data presented in Table 16.

**Table 16**

| Time of experiment | Rats' body weight, g | | |
|---|---|---|---|
| | Experimental group - control pathology 4 | Experimental group - Iodine solution + Pharmaceutical composition of HMHA | Experimental group - Iodine solution + Pharmaceutical composition of LMHA |
| Initial state | 232.50±2.81 | 194.17±3.96 | 195.17±3.96 |
| 3 days | 225.83±4.17 | 195.00±6.58 | 195.00±6.58 |
| 7 days | 225.00±5.48 | 197.50±5.74 | 200.50±5.74 |
| 14 days | 225.83±5.07 | 190.00±4.65 | 200.00±4.65 |

As a result of macroscopic studies of adhesive process development in abdominal cavity of rats, it has been established that after injury of cecal appendage caused by alcoholic iodine solution, development of the adhesive process had been observed in 100% of cases (6 out of 6) in animals from the group of Control pathology 4.

In experimental group of Iodine solution + Pharmaceutical composition, pharmaceutical composition of HMHA promotes reliable decrease in the number of animals with adhesions by 50% (in 3 out of 6) in comparison with the group of Control pathology 3, and pharmaceutical composition of LMHA - by 33% (4 out of 6) - as it is seen from data in Table 17.

**Table 17**

| Experimental group | Number of animals in experimental group | Number of animals with adhesions | Percentage of animals with adhesions, % |
|---|---|---|---|
| Control pathology 4 | 6 | 6 | 100 |
| Iodine solution + Pharmaceutical composition of HMHA | 6 | 3 | 50* |
| Iodine solution + Pharmaceutical composition of LMHA | 6 | 4 | 67 |

| | | | |
|---|---|---|---|
| Note: * - value deviation estimated using Fisher angular transformation, value significant relative to the group of Control pathology 4, p <0.05. | | | |

Score estimation of pronouncedness of adhesive process presented in Table 18 testifies to substantial antiadhesive activity of pharmaceutical composition of HMHA, as indicated by significant decrease of score points by 1.5-fold in relation to the group of Control pathology 4, and antiadhesive activity of pharmaceutical composition of LMHA by 1.2-fold.

**Table 18**

| Experimental group | Number of animals in experimental group | Pronouncedness of adhesive process, score |
|---|---|---|
| Control pathology 4 | 6 | 3.00±0.00 |
| Iodine solution + Pharmaceutical composition of HMHA | 6 | 2.00±0.44* |
| Iodine solution + Pharmaceutical composition of LMHA | 6 | 2.5±0.25 |

| | | |
|---|---|---|
| Note: * - value deviation estimated by Mann-Whitney method, value significant relative to the group of Control pathology 4, p <0.05 | | |

Determination of mass of the dissected complex of matted organs in rats after injury of cecal appendage caused by alcoholic iodine solution (see Table 19) also demonstrates that pharmaceutical composition of HMHA promotes reliable decrease of mass of the complex of matted organs by 1.7-fold in comparison with the group of Control pathology 4, and pharmaceutical composition of LMHA - by 1.3-fold, also indicating to the more pronounced antiadhesive action of the pharmaceutical composition of HMHA.

**Table 19**

| Experimental group | Mass of the complex of matted organs, g |
|---|---|
| Control pathology 4 | 1.64±0.08 |
| Iodine solution + Pharmaceutical composition of HMHA | 0.99±0.06* |
| Iodine solution + Pharmaceutical composition of LMHA | 1.25±0.05 |

| | |
|---|---|
| Note: * - value deviation estimated by Newman-Keuls method, value significant relative to the group of Control pathology 4, p <0.05 | |

Studies of macropreparations of the complex of matted organs in the location of injury of cecal appendage caused by alcoholic iodine solution demonstrate that there were observed substantial whitish thickenings at tip of the appendix and planar adhesions between its body and adjacent loop of small intestine in the group of Control pathology 4 in 100% of rats (6 out of 6).

No changes of the surface were found in 33.3% of rats from the group of Iodine solution + Pharmaceutical composition of HMHA, and in 16.7% of animals there were moderate whitish thickenings at tip of the appendix, which were combined with punctate filamentary adhesions with adjacent loop of small intestine. In 50,0% of rats marked whitish thickening at tip of the appendix and planar adhesions with loop of small intestine were observed.

Morphological studies of adhesive process development in abdominal cavity of rats after injury of cecal appendage caused by alcoholic iodine solution demonstrate that different in their pronouncedness fibrin accumulations in the form of weakly fuchsinophilic filamentary or reticular mass with sufficiently large content of cell elements were observed in all rats from the group of Control pathology 4 at the tip of vermiform appendix. Dystrophic regions are observed in muscular layer of the appendix. In the region of appendix body there are seen "soldered" strands of omentum. In some locations these adhesions had well-marked filamentary character. Similar adhesions of omentum are also found with loop of small intestine adjacent to blind intestine. Marked disintegration of muscular fibrils is determined in the locations of union, connective tissue of adhesions grows out into muscular fibrils of the intestine. After chemical injury of surface of cecal appendage with alcoholic iodine solution marked adhesive process develops - in all rats fibrin thickenings are deposited at the tip of appendix, in conjunction with development of planar adhesions between appendix body and adjacent loop of small intestine - 3 score points.

In the group of Iodine solution + Pharmaceutical composition of HMHA only small fibrin accumulations were found both at the tip and on the body of vermiform appendix in 33.3% of rats. At that muscular layer is not damaged. There were massive fibrin accumulations at tip of the appendix in 50% of animals, and strands of omentum "soldered" to the body of appendix. Sometimes they are of mature filamentary character, but nevertheless muscular layer of the appendix is virtually undamaged. Also were discovered adhesions with corresponding loop of small intestine. Disintegration of muscular fibrils takes place in locations of adhesion, but connective tissue of adhesions is not invading into muscular fibrils of intestine. Tissue of adhesions is less mature than in the group of Control pathology 4.

Therefore, it has been established as a result of studies conducted on four models of adhesive process in abdominal cavity (stitching of duodenal mesentery with silk thread, scarification of cecal appendage, application of powdered Altabor tablets to the visceral surface of spleen, and spreading of 5% alcoholic iodine solution over cecal appendage) that pharmaceutical composition of HMHA demonstrates marked antiadhesive action, decreasing the numbers of adhesions, volume of fibrin masses deposited in locations of surgical intervention, volume and extent of adhesions, and also prevents accelerated maturation of connective tissue of adhesions.

Pharmaceutical compositions according to the technical solution display wide range of therapeutic activity, have anti-inflammatory, antiseptic, regenerating, and moistening action, and may be used, in particular, as an agent to accelerate wound healing. In order to investigate the therapeutic activity of pharmaceutical compositions according to Examples 3 and 6, studies have been conducted on the pharmaceutical composition effects on wounds healing in three models of wounds - chemical burn wound, thermal burn wound, and "full-thickness skin wound".

Wound healing action of pharmaceutical compositions had been studied in the rat model of chemical burn wound caused by 20% KOH solution. Application of KOH causes actively developing hyperemia in the location of lesions, and then portion of skin was covering with necrotic crust (scab) which was drying up, getting gradually rejected and shed. The treatment was started on the next day by applying each day 0,4 g of pharmaceutical composition to the total surface of wounds in animals from the test group. Group of rats with untreated wound was used as control. Area of wounds was measured periodically in each group of animals, and histological studies of tissue portions from wound surface were conducted. Wound healing process occur under a scab and consists in drawing wound edges together, diminishing its size and closing.

In the control group, 50% of animals on day 16 still had small portions of unhealed wounds. In animals from the test group with wounds treated, process of skin regeneration proceeds substantially faster and already by day 11 a rejection of scab was observed, and on day 17 closing of wounds was noted. Average wound healing period (epithelialization) amounted to 23.5 days in the control group, and 16,4 days in experimental groups.

Histological studies of tissue from wound surface have demonstrated that picture of marked inflammatory process is observed for chemical skin burn. In the control group on day 5 vessels are dilated and filled with erythrocytes, significant hemorrhages and tissue edema under the scab, marked leukocyte infiltration. On day 13 of the experiment the inflammatory reaction was less marked. On day 18 of the experiment signs of inflammatory reaction were absent, and epithelization was observed.

In the group where wounds were treated with pharmaceutical compositions of HMHA and LMHA, greater wound healing action was observed for composition on base of LMHA - on day 5 of experiment, inflammatory process was less pronounced than in the control group of animals. Hyperemia of adjacent tissues is insignificant, no hemorrhages observed, granulation tissue appears in the wound defect, with epithelial layer forming on its edges. Already on day 10-12, new epithelial layer was covering an area of connective tissue formed in place of the skin wound defect. The results obtained demonstrate the efficiency of utilization of pharmaceutical composition on base of LMHA in the treatment of chemical burn wound, and acceleration of wound healing process.

The wound healing effect of pharmaceutical compositions on base of HMHA and LMHA was studied in the rat model of thermal burn wound caused by local (approximate area of lesions 300 mm²) action of high temperature, 250°C. The treatment and supervision of animals were carried out as in Example 1. Action of high temperature resulted in 3rd degree burn on the back skin of rats, which proceeded with suppurative inflammation phenomena and demarcation of necrotic tissue, and exfoliation of all epidermis layers being observed. The remaining deep dermal layers were swollen, with edema spreading over hypoderm. Brown-colored scab was forming overnight at the boundary between necrotic and viable tissues. The histological picture of burn surface was characterized by signs of inflammatory process.

In the control group of animals scab was drying up and rejected until day 15, with signs of inflammation being absent at day 20. Epithelial growth takes place on granulation from remaining epithelial appendages of skin and remnants of malpighian layer of epidermis, so that wounds were healed only by day 26.

In the experimental group, pharmaceutical composition on base of LMHA was displaying greater activity as compared with HMHA, the composition on base of LMHA activating wound healing process - scab was drying up and rejected until day 7, epithelialization process was completed by day 16, and wound surface was overgrown with hair covering by day 22. The results obtained testify to the efficiency of pharmaceutical composition use in the treatment of thermal burn wound, and acceleration of the wound healing process.

Wound healing effects of pharmaceutical compositions were studied in the model of "full-thickness skin wound", which was obtained in rats under general hexenal anaesthesia by dissection of skin flap with area of approximately 200 mm² in the region of anterior scapula. The treatment and supervision of animals were carried out as described in Example 1. Histological picture of the wound surface was characterized by signs of suppurative inflammation.

In the control group of animals, formation of granulation tissue, which was gradually filling up the wound defect, started only on 12^{th} day after beginning of the experiment. Planar epithelization was observed on day 21, while complete healing occurred up to day 27.

In experimental group using of pharmaceutical compositions on base of LMHA and HMHA, the composition on base of LMHA demonstrates greater activity, planar epithelization was observed in animals on day 12 after beginning of the experiment. Complete healing of wounds occurred until day 17. The results obtained testify to the efficiency of using pharmaceutical composition on base of LMHA in the treatment of wounds caused by mechanical impact on the skin, and acceleration of the wound healing process.

Thus it has been established as a result of studies in three models of wounds that pharmaceutical composition on base of LMHA demonstrates marked therapeutic activity in accelerating wound healing process of different origin.

In order to investigate effects of pharmaceutical compositions on the development of adhesions, and influence of pharmaceutical compositions on wounds healing, studies on patients have been conducted.

85 patients participated in the studies. All patients included in the studies were divided into 4 clinical groups. In each group, 2 subgroups were allocated, in which pharmaceutical compositions of HMHA and LMHA were used, and corresponding subgroups were further subdivided into three subgroups in order to study effects of pharmaceutical compositions containing hyaluronic acid od different molecular weight. Subgroups were comparable in quantitative composition, sex, age and kind of operations performed.

**Table 20. Sex distribution of patients examined**

| Group | | | Male | Female |
|---|---|---|---|---|
| Control group | LMHA n=36 | 100 kDa | 3 | 9 |
| | | 250 kDa | 6 | 6 |
| | | 400 kDa | 9 | 3 |
| | HMHA n=36 | 500 kDa | 8 | 4 |
| | | 1 million Da | 5 | 7 |
| | | 2.5 million Da | 6 | 6 |
| Group 1 | LMHA n=13 | Preparation 1 | 3 | 2 |
| | | Preparation 2 | 2 | 2 |
| | | Preparation 3 | 2 | 2 |
| | HMHA n=12 | Preparation 4 | 2 | 2 |
| | | Preparation 5 | 2 | 2 |
| | | Preparation 6 | 2 | 2 |
| Group 2 | LMHA n=12 | Preparation 1 | 2 | 2 |
| | | Preparation 2 | 2 | 2 |
| | | Preparation 3 | 2 | 2 |
| | HMHA n=12 | Preparation 4 | 2 | 2 |
| | | Preparation 5 | 2 | 2 |
| | | Preparation 6 | 2 | 2 |
| Group 3 | LMHA n=12 | Preparation 1 | - | 4 |
| | | Preparation 2 | - | 4 |
| | | Preparation 3 | - | 4 |
| | HMHA n=12 | Preparation 4 | - | 4 |
| | | Preparation 5 | - | 4 |
| | | Preparation 6 | - | 4 |

**Table 21. Age distribution of patients examined**

| | Control group | | 1^{st} group (n=25) | | 2^{nd} group (n=24) | | 3^{rd} group (n=24) | |
|---|---|---|---|---|---|---|---|---|
| Sex | LMHA n=36 | HMHA n=36 | LMHA n=13 | HMHA n=12 | LMHA n=12 | HMHA n=12 | LMHA n=12 | HMHA n=12 |
| Male | 52.5±6,3 | 54,5±5,3 | 53,8±6,6 | 56,0±6,8 | 53.7±5,2 | 58,4±2,6 | - | - |
| Female | 51,6±4, 8 | 57,5±3,3 | 59,9±2,2 | 62.5±4,8 | 60,6±5,1 | 64,1±3,7 | 27,4±3,9 | 30,4±2,0 |

The 1^{st} group comprised 38 patients undergoing planned laparotomy and resection of organs (Table 22)

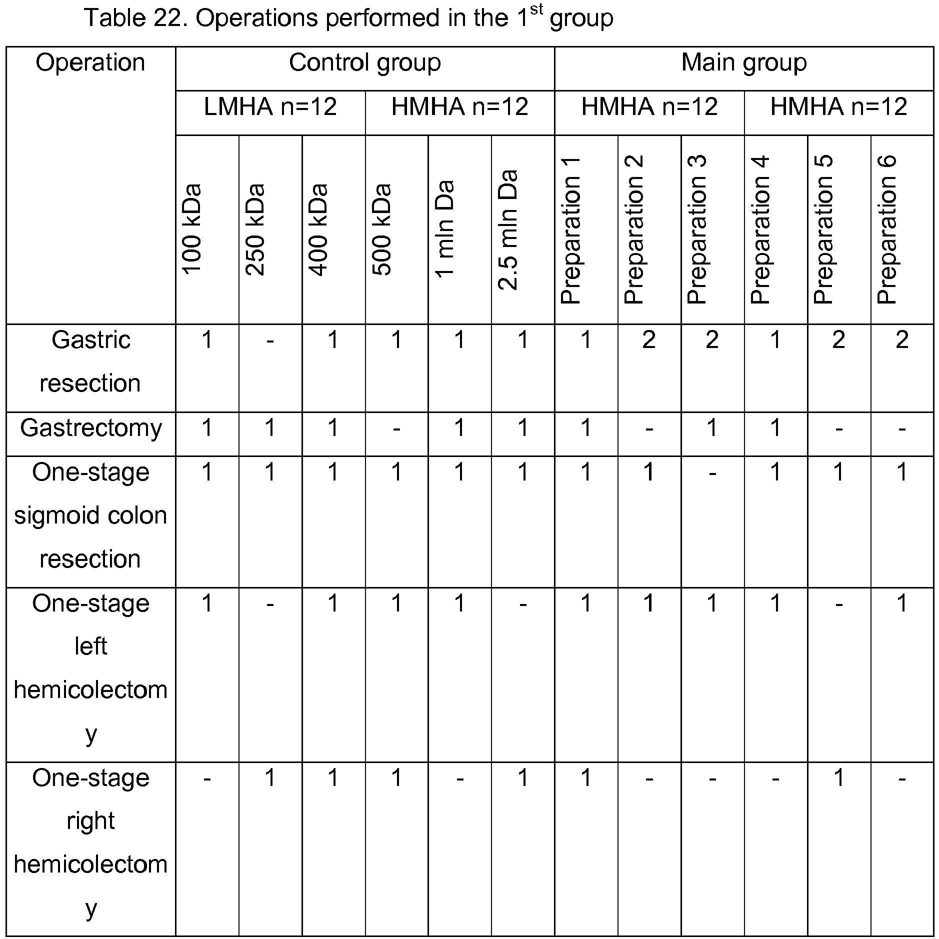

All patients were performed postoperative ultrasonic diagnostics of adhesion formation.

It is seen from data comparison in Tables 23.1 and 23.2 that adhesions of loops of small intestine with middle scar have decreased by 20-25% in the main group with preparations Nos. 1-6 administered, as opposed to the control group wherein solutions of hyaluronic acid of different molecular weight were used.

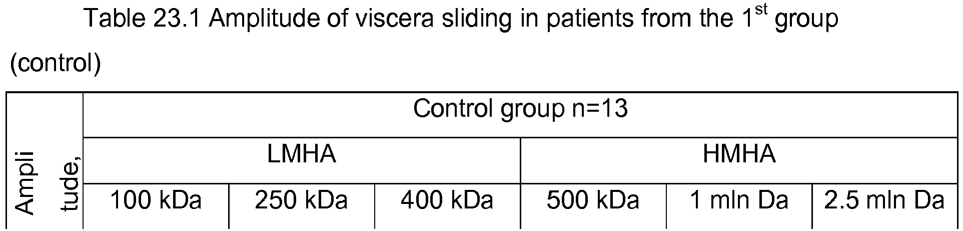

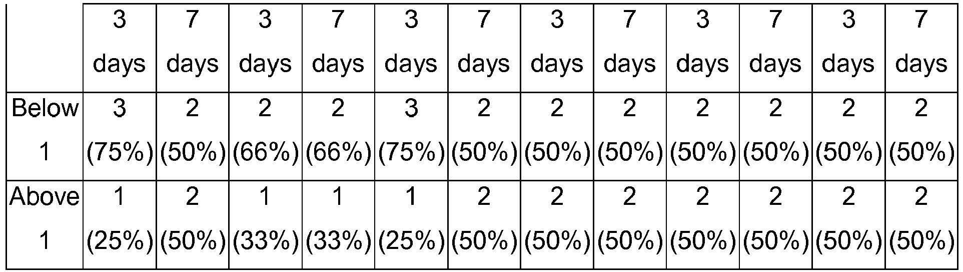

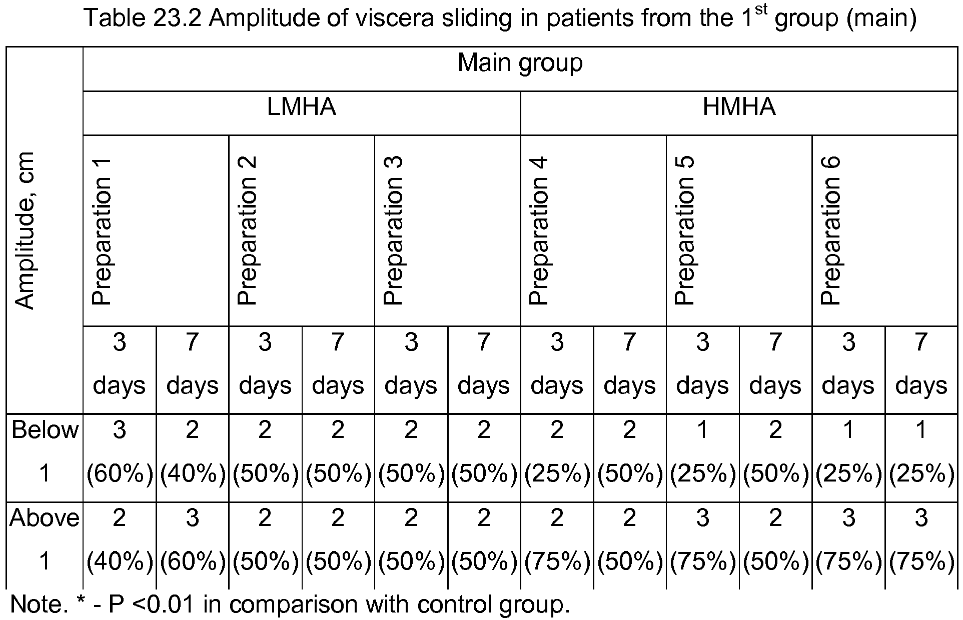

It is seen when comparing effects of preparations 1-3 and 4-6 that preparations 4-6 substantially reduce the possibility of adhesions formation, namely, by 20-25%.

However, this estimate despite its doubtless advantage of invasion absence does not allow to determine incidence of adhesive process in divisions of the abdominal cavity and macroscopic character of adhesions.

To the second group were attributed 48 patients which underwent two-stage laparotomy colorectal operation with interval exceeding 2 months (Table 24):

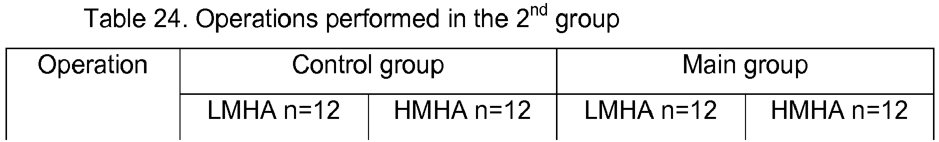

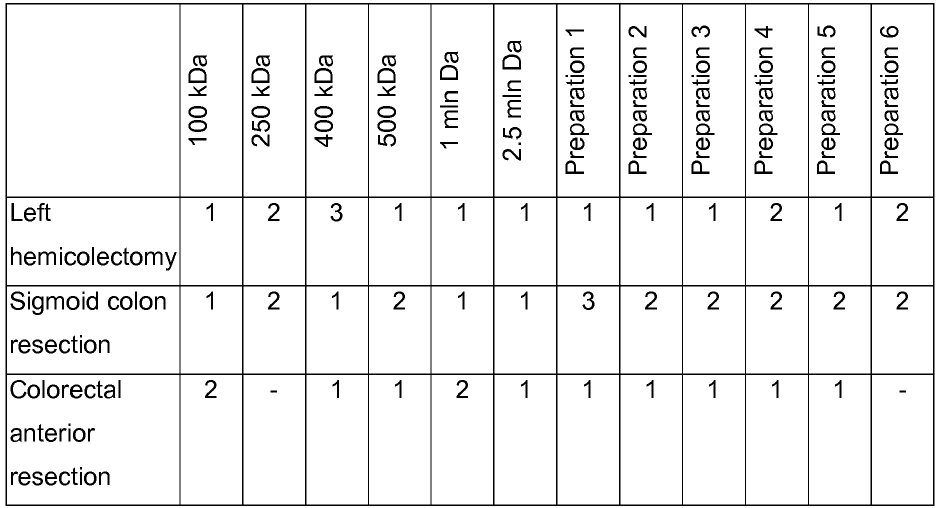

In all patients, intraoperational diagnostics of adhesive process extension was performed in the course of recurrent intervention according to questionnaire in 13 regions of peritoneum.

### Scheme of estimate for adhesive process in general surgery operations (answer)

| | Adhesions | | | | | | | |
|---|---|---|---|---|---|---|---|---|
| Anatomical region | occurrence | | | severity | | incidence | | |
| | yes | no | no region | membranous, moderate | dense, marked | <1/3 | 1/3- 2/3 | >2/3 |
| Parietal adhesions | | | | | | | | |
| Cranial anterior parietal peritoneum | | | | | | | | |
| Cranial parietal peritoneum, right | | | | | | | | |
| Cranial parietal peritoneum, left | | | | | | | | |
| Caudal anterior parietal peritoneum | | | | | | | | |
| Right pelvic wall | | | | | | | | |
| Left pelvic wall | | | | | | | | |
| Visceral adhesions | | | | | | | | |
| With large omentum | | | | | | | | |
| With small intestine | | | | | | | | |
| In subhepatic space | | | | | | | | |
| With right half of colon | | | | | | | | |
| With left half of colon | | | | | | | | |
| Close to rectosigmoid | | | | | | | | |
| In uterorectal.space | | | | | | | | |

**Table 25.1. Numerical score of adhesive process in general surgery operations in each of 13 regions**

| Adhesions | Coverage | | | | | |
|---|---|---|---|---|---|---|
| | <1/3 of region | | 1/3-2/3 of region | | >2/3 of region | |
| | planar | dense | planar | dense | planar | dense |
| 100 kDa | 5 | 6 | 6 | 6 | 5 | 3 |
| 250 kDa | 4 | 6 | 5 | 5 | 5 | 3 |
| 400 kDa | 4 | 6 | 5 | 5 | 4 | 3 |
| 500 kDa | 4 | 5 | 5 | 4 | 4 | 3 |
| 1 mln Da | 3 | 5 | 4 | 4 | 4 | 3 |
| 2.5 mln Da | 3 | 5 | 4 | 4 | 3 | 3 |

| | | | | | | |
|---|---|---|---|---|---|---|
| Note. * - No adhesions - score 0. | | | | | | |

**Table 25.2. Numerical score of adhesive process in general surgery operations in each of 13 regions**

| Adhesions | Coverage | | | | | |
|---|---|---|---|---|---|---|
| | <1/3 of region | | 1/3-2/3 of region | | >2/3 of region | |
| | planar | dense | planar | dense | planar | dense |
| Preparation 1 | 4 | 5 | 4 | 6 | 3 | 3 |
| Preparation 2 | 4 | 5 | 4 | 5 | 3 | 3 |
| Preparation 3 | 4 | 5 | 3 | 5 | 3 | 3 |
| Preparation 4 | 3 | 4 | 3 | 4 | 3 | 2 |
| Preparation 5 | 2 | 4 | 3 | 4 | 3 | 2 |
| Preparation 6 | 1 | 4 | 3 | 3 | 2 | 2 |

| | | | | | | |
|---|---|---|---|---|---|---|
| Note. * - No adhesions - score 0. | | | | | | |

It is seen from the above Tables 25.1 and 25.2 that use of pharmaceutical compositions (preparations 1-6) results in reduction of adhesions formation by 76%.

The third group comprised 48 patients which underwent laparoscopy operations on uterine appendages concerning tubo-peritoneal infertility with 2 months interval (Table 26).

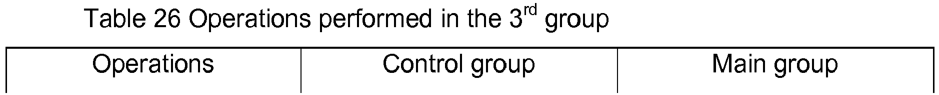

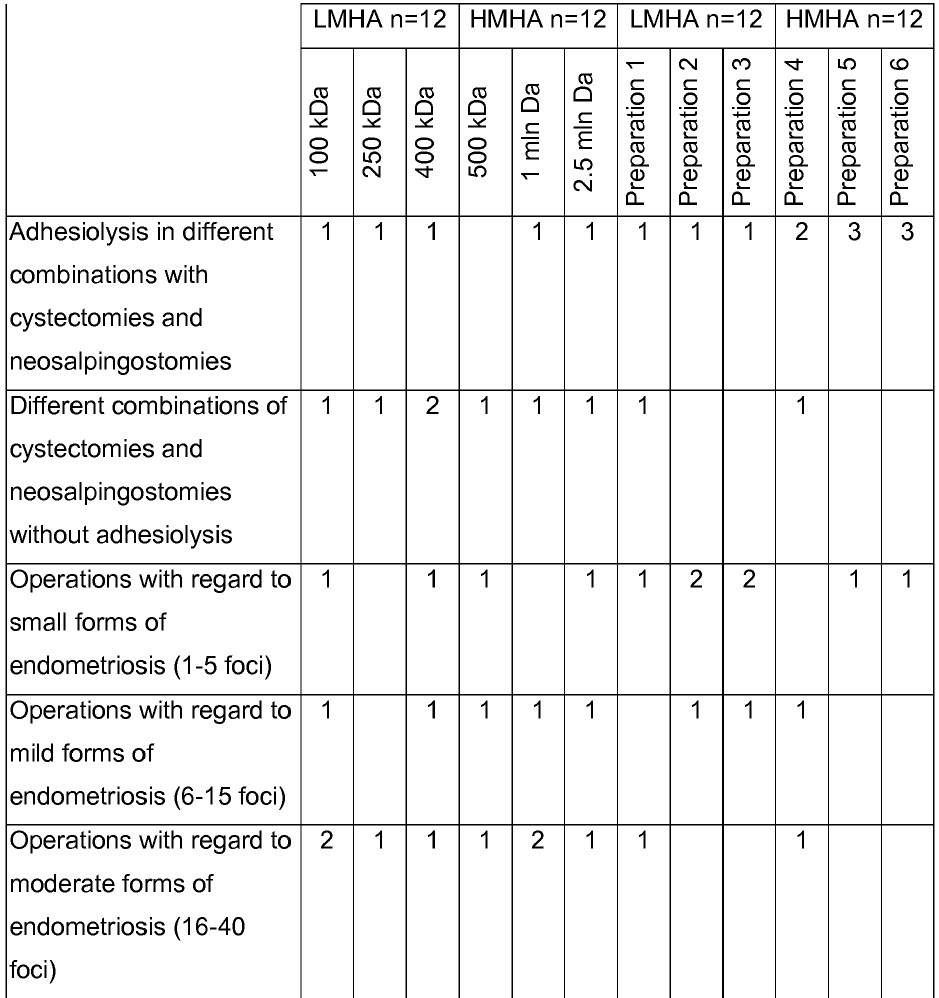

Estimate of adhesive process was performed in the course of primary laparoscopy according to the AFS score system (American Fertility Society). Secondary adhesions formation was evaluated in the course of re-laparoscopy or culdoscopy, also according to the AFS score system.

Classification of American Fertility Society (1979) discovers 4 severity grades of the disease depending on the number and size of adhesive lesions foci (American Fertility Society. Classification of endometriosis // Fertil. Steril. - 1979, Vol. 32, Nº5-6, 633-634) :
I. presence of 1-5 adhesions - rated as mild grade
II. 6-15 - as moderate
III. 15-30 - as severe
IV. more than 30 foci is rated as extensive endometriosis

Based on this classification, numerical score system for grades of adhesive process extension (Table 27) was published in 1985.

**Table 27**

| Adhesions (volume of damage tissue) | | | < 1/3 adhesed | 1/3 - 2/3 adhesed | > 2/3 adhesed |
|---|---|---|---|---|---|
| Ovaries | Right | Delicate | 1 | 2 | 4 |
| | | Dense | 4 | 8 | 16 |
| | Left | Delicate | 1 | 2 | 4 |
| | | Dense | 4 | 8 | 16 |
| Tubes | Right | Delicate | 1 | 2 | 4 |
| | | Dense | 4* | 8* | 16 |
| | Left | Delicate | 1 | 2 | 4 |
| | | Dense | 4* | 8* | 16 |

| | | | | | |
|---|---|---|---|---|---|
| * Completely adhesed fimbrial department of tubes should be assessed as ">16" | | | | | |

**Table 28.1. Numerical score of adhesive process in general surgery operations in each of 13 regions (group 3 control)**

| Adhesions | Coverage | | | | | |
|---|---|---|---|---|---|---|
| | <1/3 of region | | 1/3-2/3 of region | | >2/3 of region | |
| | planar | dense | planar | dense | planar | dense |
| 100 kDa | 5 | 6 | 6 | 6 | 5 | 3 |
| 250 kDa | 4 | 6 | 5 | 5 | 5 | 3 |
| 400 kDa | 4 | 6 | 5 | 5 | 4 | 3 |
| 500 kDa | 4 | 5 | 5 | 4 | 4 | 3 |
| 1 min Da | 3 | 5 | 4 | 4 | 4 | 3 |
| 2.5 min Da | 3 | 5 | 4 | 4 | 3 | 3 |

| | | | | | | |
|---|---|---|---|---|---|---|
| *Note.* * - *No adhesions - score 0.* | | | | | | |

**Table 28.2. Numerical score of adhesive process in general surgery operations in each of 13 regions (group 3 main)**

| Adhesions | Coverage | | | | | |
|---|---|---|---|---|---|---|
| | <1/3 of region | | 1/3-2/3 of region | | >2/3 of region | |
| | planar | dense | planar | dense | planar | dense |
| Preparation 1 | 4 | 5 | 4 | 6 | 3 | 3 |
| Preparation 2 | 4 | 5 | 4 | 5 | 3 | 3 |
| Preparation 3 | 4 | 5 | 3 | 5 | 3 | 3 |
| Preparation 4 | 3 | 4 | 3 | 4 | 3 | 2 |
| Preparation 5 | 2 | 4 | 3 | 4 | 3 | 2 |
| Preparation 6 | 1 | 4 | 3 | 3 | 2 | 2 |

| | | | | | | |
|---|---|---|---|---|---|---|
| Note. * - No adhesions - score 0. | | | | | | |

According to the AFS scale, it is seen from the data in Tables 28.1 and 28.2 that severity of adhesive process extension in small pelvis was reduced by using preparations 1-6 on the average by 76%, correspondingly.

Transvaginal ultrasound scan (TVUS) of gynaecological patients has revealed no: 1) blurring of ovary boundaries, which is defined as the absence of sharp ovary outline over more than 3/4 of its extent; 2) attachment of ovary to the uterus which persisted in abdominal palpation; 3) increase in destance from ovary to the sensor over 11 mm which persisted in abdominal palpation.

24 patients participated in the studies of preparations 1-6 effects on wound healing. Patients had different superficial injuries (wounds bottoms were represented by muscular tissue). Patients were divided into 12 groups of 2 people each, which were prescribed preparations 1-6 and hyaluronic acid of different molecular weight, correspondingly.

In the first day after application of preparations 1-6, macroscopic borders of wounds were moderately infiltrated, swelling and hyperemia of cutaneous cover around wounds remained unchanged, surface wound defect was covered with fibrinous film; exudate of a seropurulent character was in moderate quantities. On the second day, size of the wound defect was reduced on the average by 30%, infiltration of wounds margins remained, insignificant amount of serous exudate; flabby granulations appear on the bottom of wounds. On the 3-4 days, inflammatory symptoms are relieved, and active granulation begins.

**Table 30. Rate of wound healing**

| | Time, days | | |
|---|---|---|---|
| | Appearance of granulations | Beginning of epithelialization | Healing |
| 100 kDa | 3 | 5 | 11 |
| 250 kDa | 4 | 6 | 11 |
| 400 kDa | 4 | 7 | 12 |
| 500 kDa | 5 | 7 | 13 |
| 1 mln Da | 5.5 | 8 | 13 |
| 2.5 mln Da | 6 | 9 | 14 |
| Preparation 1 | 2.5 | 4.5 | 9.5 |
| Preparation 2 | 3 | 5 | 11 |
| Preparation 3 | 4 | 5.5 | 11 |
| Preparation 4 | 4 | 6 | 11 |
| Preparation 5 | 5 | 6 | 11 |
| Preparation 6 | 5 | 6 | 11 |

As it is seen from Table 30, preparations 1-6 promote early healing of wounds due to decamethoxine present in composition, thus providing for disinfection of surface wounds and creating possibility for epithelium to recover at faster rate.

Studies were also conducted of pharmaceutical compositions HA + decamethoxine.

In comparative studies of efficiency and tolerance of pharmaceutical composition of HA with decamethoxine in acute vaginal infections took part 90 women (45 with diagnosis of bacterial vaginosis, and 45 with vaginal candidiasis). 50 patients were prescribed pharmaceutical composition of HA with decamethoxine (5 ml 1-2 times daily), 25 patients - 0.5% pharmaceutical composition of HA with chlorhexidine (2.5 g/day), and 15 patients - 2% pharmaceutical composition of HA with metronidazole once daily. The duration of the studies is 4 weeks.

In bacterial vaginosis clinical treatment was observed in 90% of patients administered pharmaceutical composition of HA with decamethoxine, in comparison with 75% in the group of patients administered pharmaceutical composition of HA with chlorhexidine; also in case of using the pharmaceutical composition of HA with decamethoxine there was noted a significant decrease in vaginal pH up to 3 weeks of the study (in case of chlorhexidine - up to 4 weeks). In vaginal candidiasis clinical treatment was observed in 85% of patients administered pharmaceutical composition of HA with decamethoxine, in comparison with 80% in the group of women administered pharmaceutical composition of HA with chlorhexidine.

No serious side effects were revealed in studies of pharmaceutical composition of HA with decamethoxine. Tolerance of the pharmaceutical composition was estimated as "very good" by the majority (90%) of patients.

Thus, in bacterial vaginosis and vaginal candidiasis the pharmaceutical composition of HA with decamethoxine has demonstrated its efficiency, and short therapy course using this pharmaceutical composition is equivalent in efficiency to standard techniques.

To investigate bioadhesive capacity of pharmaceutical composition of HA with decamethoxine foor use in medicine, following studies were conducted:

Course of deliveries was analyzed in 120 women. Main group (first group) comprised 60 patients with deliveries carried out using pharmaceutical composition of HA with decamethoxine and hyaluronic acid. Control group (second group) comprised 60 patients which don't used said gel in the course of deliveries.

Average age of women in childbirth in the main group was 28 years, and in control group - 29 years. It was found that number of firstborns in the main group amounted to 30 (50%), and that in the control group - 32 (53.3%).

Second delivery took place in the main group in 25 (41.6%) women, and in 20 (33.3%) - in the control group.

Third and more deliveries were noted in 5 (8.3%) women in the main group, and in 8 (13.3%) in the comtrol group.

Injuries of birth canal soft tissues had in anamnesis 20 (33.3%) patients from the main group and 15 (25%) from the control one; lacerations of cervix were in anamnesis in 5 (8.3%) women from the main and in 5 (8.3%) from control group; and perineum traumas - in 12 (20%) and 10 (16,6%) parturient women, correspondingly.

All women both in the main and control groups were admitted to the obstetric department in the first period of childbirth. Monitoring supervision over the fetus state and uterine contractile activity was conducted throughout the delivery period.

Pharmaceutical composition of HA with decamethoxine was used in delivery during each vaginal examination, beginning from the first one: 5 ml of gel was distributed uniformly over the vaginal birth canal. Additional application of gel was performed 15-30 minutes after breaking of waters or amniotomy.

### Results:

Analysis has demonstrated that vaginal traumatism took place in 5 (8.3%) women of the main group and in 10 (16,6%) from the control group. Lacerations of cervix were found in 3 (5%) patients from the 1^{st} group, and in 15 (25%) from the control one. Episiotomy was performed in 2 cases (3.3%) in parturient women from the main group (large fetus) and in 3 (5%) cases in the control group (large fetus threatening with perineal tear by the old scar).

Pain was estimated as slight by 35 (58.3%) parturient women from the main, and by 15 (25%) from control groups; as moderate - by 36 (60%) in the main group, and by 32 (53.3%) in the control one. Complaints of the pronounced pain were made by 5 (8.3%) women patients from the 2^{nd} group. There were no complaints of the pronounced pain in childbirth in the main group.

Thus, use of pharmaceutical composition of HA with decamethoxine in childbirth management allows to reduce the number of cases of soft tissues traumatism in genital tracts, to decrease duration of labor and to diminish incidence of operative delivery. Besides, decrease in pain syndrome had been noted in all patients of the control group, and by subjective sensation of women course of delivery was substantially relieved.

### Advantages of pharmaceutical composition:

Pharmaceutical composition displays potent bioadhesive properties and high water-binding capacity. These properties of the pharmaceutical composition are provides by hyaluronic acid present in pharmaceutical composition (HA binds and retains water. One its molecule binds up to a thosand of water molecules).

As a result, this pharmaceutical composition forms low-viscous bioadhesive film in vaginal birth canal, thus reducing friction between the same and the child which could result in complications in the delivery process. This film maintains natural humidity of the skin without disturbing gaseous exchange.

Also one of the principal advantages of hyaluronic acid is the fact of HA being natural component of our body, and therefore is perfectly compatible with skin and causes no allergic reactions and irritations.

**Table 31. Comparison of antimicrobial activity for pharmaceutical composition of HA with decamethoxine**

| Causative agents | Pharmaceutical composition of HA with decamethoxine | Pharmaceutical composition of HA with chlorhexidine | Pharmaceutical composition of HA with metronidazole | Control |
|---|---|---|---|---|
| *Candida albicans* | ++ | + | + | +++ |
| *Gardnerella vaginalis* | +++ | + | + | +++ |
| *Bacteroides urealyticus* | ++ | + | + | +++ |
| *Trichomonas vaginalis* | + | | + | +++ |
| *Chlamydia trachomatis* | + | | + | +++ |
| *Haemophilus influenza* | ++ | | + | +++ |
| *Enterococcus faecalis* | +++ | ++ | ++ | +++ |
| *Streptococcus agalactiae* | +++ | + | ++ | +++ |
| *Streptococcus pyogenes* | +++ | ++ | ++ | +++ |
| *Staphylococcus sepidermidis* | +++ | ++ | ++ | +++ |
| *Staphylococcus aureus* | +++ | ++ | ++ | +++ |

| | | | | |
|---|---|---|---|---|
| "-" no activity; "+" low activity; "++" good activity; "+++" high activity | | | | |

Conclusion: as a result of comparative studies, the pharmaceutical composition of HA with decamethoxine displays bactericidal effect in relation to anaerobic bacteria, and bacteriostatic in relation to protozoa. It is active in relation to most strains of microorganisms causing bacterial vaginosis: Gardnerella vaginalis, Bacteroides spp., Mobiluncus spp., Peptostreptococcus spp.

To investigate pharmaceutical compositions, many modern studies were used, although only part of them is presented in the specification, which should demonstrate achievement of the objectives stated without limiting in any way the technical solution.

It is seen from the studies presented that pharmaceutical compositions on base of stabilized solution of hyaluronic acid and/or its pharmaceutically acceptable salts together with decamethoxine and/or its water-soluble salts are effective both in prevention of adhesions formation and in provoding wound-healing, bactericidal effects. Pharmaceutical compositions with HMHA and decamethoxine are in greater extent aimed at the prevention adhesions formation than LMHA, however LMHA is more effective in relation to infections in different parts of the body. Therefore, in some cases such compositions may be used separately, Otherwise, they may be used together, since effective combination of such compositions may embrace total range of action of the preparation from vaginal infections, childbirth, operative treatment, to prevention of adhesion formation.

It is seen from the above that technical result is preparation of stabilized solution comprising decamethoxine and hyaluronic acid or its pharmaceutically acceptable salt, without precipitation in solution, which display anti-inflammatory, antiseptic, regenerating and moistening effects, in particular, demonstrate therapeutic activity in regard to suppression of adhesive process, and preparation of pharmaceutical composition in a pharmaceutically acceptable dosage form on base of this stabilized solution providing therapeutic activity, in particular, providing pronounced therapeutic action by accelerating the process of healing wounds of different origin, and also broadening the range of medicinal agents possessing therapeutic activity in regard to suppression of adhesive process and activity in accelerating processes of healing wounds of different origin.

The examples of embodiments provided illustrate technical solution without limiting its scope.

## Claims

1. A stabilized solution on base of two active substances exhibiting therapeutic activity, ***characterized in that*** it further contains stabilizer, hyaluronic acid and/or its pharmaceutically acceptable salt is used as first active substance, decamethoxine and/or its water-soluble salt is used as second active substance, stabilizer is a pharmaceutically acceptable salt capable of dissolving in water and dissotiating in aqueous solutions into metal cations and acid residues anions, or mixture of such salts, the stabilizer being capable at the same time to form water-soluble compounds with hyaluronic acid and/or its pharmaceutically acceptable salt and with decamethoxine and/or its water-soluble salt.

2. The stabilized solution according to claim 1, ***characterized in that*** the stabilizer is a nontoxic salt or low toxic salt of inorganic acids, organic mono- and dicarboxylic acids, or mixture of such salts.

3. The stabilized solution according to claim 2, ***characterized in that*** the stabilizer is sodium chloride, sodium succinate, sodium lactate, zinc chloride, zinc sulphate or any their mixture.

4. The stabilized solution according to claim 1, ***characterized in that*** it comprises hyaluronic acid and/or its pharmaceutically acceptable salt, decamethoxine and/or its water-soluble salt, stabilizer and water, at the following components ratio, mg/ml:
| | |
|---|---|
| hyaluronic acid and/or its pharmaceutically acceptable salt | 1-50 |
| decamethoxine and/or its water-soluble salt | 0.01-10 |
| stabilizer | 1-60 |
| water | q.s. to 1 ml. |

5. The stabilized solution according to any of claims 1-3, ***characterized in that*** it comprises hyaluronic acid and/or its pharmaceutically acceptable salt, decamethoxine and/or its water-soluble salt, stabilizer and water, and further contains at least one pharmaceutically acceptable additive, at the following components ratio, mg/ml:
| | |
|---|---|
| hyaluronic acid and/or its pharmaceutically acceptable salt | 1-50 |
| decamethoxine and/or its water-soluble salt | 0.01-10 |
| | |
|---|---|
| stabilizer | 1-60 |
| pharmaceutically acceptable additive | 1-60 |
| water | q.s. to 1 ml. |

6. The stabilized solution according to claim 5, ***characterized in that*** it comprises as pharmaceutically acceptable additive an agent to maintain pH of the solution at a constant level.

7. The stabilized solution according to claim 6, ***characterized in that*** it comprises sodium succinate as stabilizer and succinic acid as an agent to maintain pH of the solution at a constant level.

8. The stabilized solution according to claim 6, ***characterized in that*** it comprises sodium lactate as stabilizer and lactic acid as an agent to maintain pH of the solution at a constant level.

9. The stabilized solution according to any of claims 5-7, ***characterized in that*** it is an aqueous solution comprising hyaluronic acid and/or its pharmaceutically acceptable salt, sodium succinate, succinic acid, sodium chloride, decamethoxine and/or its water-soluble salt, and water, at the following components ratio, mg/ml:
| | |
|---|---|
| hyaluronic acid and/or its pharmaceutically acceptable salt | 5 |
| sodium succinate | 16 |
| succinic acid | 0.05 |
| sodium chloride | 1.2 |
| decamethoxine and/or its water-soluble salt | 0.2 |
| water | q.s. to 1 ml. |

10. A method for preparing a the stabilized solution according to claim 1, ***characterized in that*** one of active substances and stabilizer are dissolved in water, and then second active substance is dissolved in the solution obtained with mixing until its complete dissolution, hyaluronic acid and/or its pharmaceutically acceptable salt and decamethoxine and/or its water-soluble salt being used as active substances, and stabilizer is a pharmaceutically acceptable salt capable of dissolving in water and dissotiating in aqueous solutions into metal cations and acid residues anions, or mixture of such salts, the stabilizer being capable to form at the same time water-soluble compounds with hyaluronic acid and/or its pharmaceutically acceptable salt and with decamethoxine and/or its water-soluble salt.

11. The method according to claim 10, ***characterized in that*** the stabilizer is dissolved in water, and then decamethoxine and/or its water-soluble salt is dissolved, after which hyaluronic acid and/or its pharmaceutically acceptable salt is dissolved in the solution obtained.

12. The method according to claim 10, ***characterized in that*** decamethoxine and/or its water-soluble salt is dissolved in water, and then stabilizer is dissolved, after which hyaluronic acid and/or its pharmaceutically acceptable salt is dissolved in the solution obtained.

13. The method according to claim 10, ***characterized in that*** the stabilizer is dissolved in water, and then hyaluronic acid and/or its pharmaceutically acceptable salt is dissolved, after which decamethoxine and/or its water-soluble salt is dissolved in the solution obtained.

14. The method according to claim 10, ***characterized in that*** hyaluronic acid and/or its pharmaceutically acceptable salt is dissolved in water, and then stabilizer is dissolved, after which decamethoxine and/or its water-soluble salt is dissolved in the solution obtained.

15. The method according to any of claims 10-14, ***characterized in that*** the pH-stabilized solution is further adjusted to the desired value by adding acid or base.

16. The method according to any of claims 10-15, ***characterized in that*** the stabilized solution is additionally sterilized by autoclaving.

17. The method according to claim 10, ***characterized in that*** the components are used in following ratio, mg/ml:
| | |
|---|---|
| hyaluronic acid and/or its pharmaceutically acceptable salt with molecular weight from 401 kDa to 2.5 MDa | 1-50 |
| decamethoxine and/or its water-soluble salt | 0.01-10 |
| stabilizer | 1-60 |
| pharmaceutically acceptable additive | 1-60 |
| water | q.s. to 1 ml. |

18. A pharmaceutical composition on base of stabilized solution of two active substances, ***characterized in that*** it contains as first active substance high-molecular hyaluronic acid and/or its pharmaceutically acceptable salt, the molecular weight of such acid or salts may be from 401 kDa to 2, 5 MDa, and contains as second active substance decamethoxine and/or its water-soluble salt, stabilizer, which is a pharmaceutically acceptable salt capable of dissolving in water and dissotiating in aqueous solutions into metal cations and acid residues anions, or mixture of such salts, the stabilizer being capable to form at the same time water-soluble compounds with hyaluronic acid and/or its pharmaceutically acceptable salt and with decamethoxine and/or its water-soluble salt, and also contains at least one pharmaceutically acceptable additive.

19. The pharmaceutical composition according to claim 18, ***characterized in that*** the composition comprises components in the following ratio, mg/ml:
| | |
|---|---|
| hyaluronic acid and/or its pharmaceutically acceptable salt with molecular weight from 401 kDa to 2.5 MDa | 1-50 |
| decamethoxine and/or its water-soluble salt | 0.01-10 |
| stabilizer | 1-60 |
| pharmaceutically acceptable additive | 1-60 |
| water | q.s. to 1 ml. |

20. The pharmaceutical composition according to claim 19, ***characterized in that*** the stabilizer is a nontoxic salt or low toxic salt of inorganic acid, organic monocarboxylic or dicarboxylic acid, or any mixture of such salts.

21. The pharmaceutical composition according to claim 20, ***characterized in that*** the stabilizer is sodium chloride, sodium succinate, sodium lactate, zinc chloride, zinc sulphate, or any mixture of these salts.

22. The pharmaceutical composition according to claim 21, ***characterized in that*** the pharmaceutically acceptable salt of high-molecular hyaluronic acid is sodium hyaluronate.

23. The pharmaceutical composition according to claim 19, ***characterized in that*** the composition comprises as pharmaceutically acceptable additive pH adjustment agent or buffer.

24. The pharmaceutical composition according to claim 23, ***characterized in that*** the buffer comprises phosphate buffer, or citrate buffer, or acetate buffer, or succinate buffer, or tris hydrochloride buffer, or maleate buffer.

25. The pharmaceutical composition according to claim 23, ***characterized in that*** the composition comprises as pH adjustment agent a substance from the series of hydrochloric acid, sulfuric acid, nitric acid, phosphoric acid, acetic acid, propionic acid, hydroxyacetic acid, lactic acid, pyruvic acid, oxalic acid, malonic acid, succinic acid, maleic acid, fumaric acid, tartaric acid, citric acid, ascorbic acid, ammonium acetate, ammonia solution, alkaline-earth metal hydroxide, alkali metal hydroxide, sodium carbonate, sodium hydrocarbonate, and sodium phosphate.

26. The pharmaceutical composition according to claim 23, ***characterized in that*** the composition comprises sodium succinate as stabilizer, and comprises succinic acid as pH adjustment agent.

27. The pharmaceutical composition according to claim 23, ***characterized in that*** the composition comprises as sodium lactate stabilizer, and comprises lactic acid as an agent for adjusting the solution pH.

28. The pharmaceutical composition according to claim 19, ***characterized in that*** the composition is used to suppress adhesive process.

29. The pharmaceutical composition according to claim 19, ***characterized in that*** the composition is used to protect organs against adhesions formation and to prevent dehydration of organs with simultaneous anti-inflammatory effect.

30. The pharmaceutical composition according to claim 19, ***characterized in that*** the composition comprises at least one useful auxiliary pharmaceutical component as pharmaceutically acceptable additive in order to convert it into dosage form from soft to liquid.

31. The pharmaceutical composition according to claim 30, ***characterized in that*** the dosage form is aerosol, suspension, syrup, drops, solution for injection, cream, gel, ointment, paste, liniment, solution for external use, solution for oral application.

32. The pharmaceutical composition according to claim 30, ***characterized in that*** the composition comprises as pharmaceutically acceptable additive at least one auxiliary pharmaceutical component from the series of viscosity regulator, preservative, and antioxidant.

33. The pharmaceutical composition according to claim 32, ***characterized in that*** the composition comprises as viscosity regulator a substance from the series of:alkylcellulose derivative, hydroxyalkylcellulose derivative, methylcellulose, hydroxypropyl methylcellulose, hydroxybutylcellulose, carboxymethylcellulose, starch, xanthane gum, guar gum, polyacrylic acids and their salts, copolymers of methacrylate, polyethylene oxide, polypropylene oxide, chitin derivatives, starch, pectin, alginic acid, cyclodextrin, agar, carrageenan.

34. The pharmaceutical composition according to claim 32, ***characterized in that*** the composition comprises as preservative a substance from the series of:
lauralkonium chloride, benzalkonium chloride, benzododecinium chloride, cetylpyridinium chloride, cetrimide, domiphen bromide, benzyl alcohol, chlorobutanol, o-cresol, chlorocresol, phenol, ethylphenyl alcohol, benzoic acid, sodium benzoate, sorbic acid, potassium sorbate, paraaminobenzoic acid, methyl parahydroxybenzoate, propyl parahydroxybenzoate.

35. The pharmaceutical composition according to claim 32, ***characterized in that*** the composition comprises as antioxidant a substance from the series of sodium metabisulfite, sodium bisulfite, sodium sulfite, sodium thiosulphate, and ascorbic acid.

36. The pharmaceutical composition according to claim 31, ***characterized in that*** the dosage form is gel comprising high-molecular hyaluronic acid and/or its pharmaceutically acceptable salt with molecular weight from 401 kDa to 2.5 million Da, decamethoxine and/or its water-soluble salt, sodium lactate, lactic acid and water, and comprises as auxiliary pharmaceutical components glycerol and hydroxypropyl methylcellulose, at the following components ratio, mg/ml:
| | |
|---|---|
| high-molecular hyaluronic acid and/or its pharmaceutically acceptable salt with molecular weight from 401 kDa to 2.5 million Da | 2-8 |
| sodium lactate | 0.5-3 |
| lactic acid | 0.1-1 |
| decamethoxine and/or its water-soluble salt | 0.05-0.5 |
| glycerol | 20-50 |
| hydroxypropyl methylcellulose | 15-50 |
| water | q.s. to 1 ml. |

37. The pharmaceutical composition according to claim 31, ***characterized in that*** the dosage form is a gel comprising high-molecular hyaluronic acid and/or its pharmaceutically acceptable salt with molecular weight from 401 kDa to 2.5 million Da, decamethoxine and/or its water-soluble salt, sodium lactate, lactic acid and water, and comprises as auxiliary pharmaceutical components glycerol and hydroxypropyl methylcellulose, at the following components ratio, mg/ml:
| | |
|---|---|
| high-molecular hyaluronic acid with molecular weight from 401 kDa to 2.5 million Da | 5 |
| sodium lactate | 1.42 |
| lactic acid | 0.36 |
| decamethoxine and/or its water-soluble salt | 0.2 |
| glycerol | 30 |
| hydroxypropyl methylcellulose | 30 |
| water | q.s. to 1 ml. |

38. Pharmaceutical composition on base of stabilized solution of two active substances, ***characterized in that*** the stabilized solution of two active substances comprises as first active substance low-molecular hyaluronic acid and/or its pharmaceutically acceptable salt, the molecular weight of such acid or salts may be from 100 kDa to 400 kDa, comprises as second active substance decamethoxine and/or its water-soluble salt, stabilizer, which is a pharmaceutically acceptable salt capable of dissolving in water and dissotiating in aqueous solutions into metal cations and acid residues anions, or mixture of such salts, the stabilizer being capable to form water-soluble compounds at the same time with hyaluronic acid and/or its pharmaceutically acceptable salt and with decamethoxine and/or its water-soluble salt, and also contains at least one pharmaceutically acceptable additive.

39. The pharmaceutical composition according to claim 38, ***characterized in that*** the composition comprises components in the following ratio, mg/ml:
| | |
|---|---|
| hyaluronic acid and/or its pharmaceutically acceptable salt with molecular weight from 100 kDa to 400 kDa | 1-50 |
| decamethoxine and/or its water-soluble salt | 0.01-10 |
| stabilizer | 1-60 |
| pharmaceutically acceptable additive | 1-60 |
| water | q.s. to 1 ml |

40. The pharmaceutical composition according to claim 38, ***characterized in that*** the stabilizer is a nontoxic salt or low toxic salt of inorganic acid, organic monocarboxylic or dicarboxylic acid, or any mixture of such salts.

41. The pharmaceutical composition according to claim 38, ***characterized in that*** the stabilizer is sodium chloride, sodium succinate, sodium lactate, zinc chloride, zinc sulphate, or any mixture of these salts.

42. The pharmaceutical composition according to claim 38, ***characterized in that*** the pharmaceutically acceptable salt of low-molecular hyaluronic acid is sodium hyaluronate.

43. The pharmaceutical composition according to claim 38, ***characterized in that*** the composition comprises as pharmaceutically acceptable additive a pH adjustment agent or buffer.

44. The pharmaceutical composition according to claim 43, ***characterized in that*** the buffer comprises phosphate buffer, or citrate buffer, or acetate buffer, or succinate buffer, or tris hydrochloride buffer, or maleate buffer.

45. The pharmaceutical composition according to claim 43, ***characterized in that*** the composition comprises as pH adjustment agent a substance from the series of hydrochloric acid, sulfuric acid, nitric acid, phosphoric acid, acetic acid, propionic acid, hydroxyacetic acid, lactic acid, pyruvic acid, oxalic acid, malonic acid, succinic acid, maleic acid, fumaric acid, tartaric acid, citric acid, ascorbic acid, ammonium acetate, ammonia solution, alkaline-earth metal hydroxide, alkali metal hydroxide, sodium carbonate, sodium hydrocarbonate, and sodium phosphate.

46. The pharmaceutical composition according to claim 43, ***characterized in that*** the composition comprises sodium succinate as stabilizer, and comprises succinic acid as pH adjustment agent.

47. The pharmaceutical composition according to claim 43, ***characterized in that*** the composition comprises sodium lactate as stabilizer, and comprises lactic acid as an agent for adjusting the solution pH.

48. The pharmaceutical composition according to claim 38, ***characterized in that*** the composition is used to suppress adhesive process.

49. The pharmaceutical composition according to claim 38, ***characterized in that*** the composition is used to protect organs against adhesions formation and to provide wound healing with simultaneous anti-inflammatory effect.

50. The pharmaceutical composition according to claim 38, ***characterized in that*** the composition comprises as pharmaceutically acceptable additive at least one useful auxiliary pharmaceutical component to convert it into dosage form from soft to liquid.

51. The pharmaceutical composition according to claim 50, ***characterized in that*** the dosage form is aerosol, suspension, syrup, drops, solution for injection, cream, gel, ointment, paste, liniment, solution for external use, solution for oral application.

52. The pharmaceutical composition according to claim 50, ***characterized in that*** the composition comprises as pharmaceutically acceptable additive at least one auxiliary pharmaceutical component from the series of: viscosity regulator, preservative, and antioxidant.

53. The pharmaceutical composition according to claim 52, ***characterized in that*** the composition comprises as viscosity regulator a substance from the series of:
alkylcellulose derivative, hydroxyalkylcellulose derivative, methylcellulose, hydroxypropyl methylcellulose, hydroxybutyl cellulose, carboxymethylcellulose, starch, xanthane gum, guar gum, polyacrylic acids and their salts, copolymers of methacrylate, polyethylene oxide, polypropylene oxide, chitin derivatives, starch, pectin, alginic acid, cyclodextrin, agar, and carrageenan.

54. The pharmaceutical composition according to claim 52, ***characterized in that*** the composition comprises as preservative a substance from the series of:
lauralkonium chloride, benzalkonium chloride, benzododecinium chloride, cetylpyridinium chloride, cetrimide, domiphen bromide, benzyl alcohol, chlorobutanol, o-cresol, chlorocresol, phenol, ethylphenyl alcohol, benzoic acid, sodium benzoate, sorbic acid, potassium sorbate, paraaminobenzoic acid, methyl parahydroxybenzoate, propyl parahydroxybenzoate.

55. The pharmaceutical composition according to claim 52, ***characterized in that*** the composition comprises as antioxidant a substance from the series of: sodium metabisulfite, sodium bisulfite, sodium sulfite, sodium thiosulphate, and ascorbic acid.

56. The pharmaceutical composition according to claim 51, ***characterized in that*** the dosage form is a gel containing low-molecular hyaluronic acid and/or its pharmaceutically acceptable salt with molecular weight from 100 kDa to 400 kDa, decamethoxine and/or its water-soluble salt, sodium lactate, lactic acid and water, and containiing as auxiliary pharmaceutical components glycerol and hydroxypropyl methylcellulose, at the following components ratio, mg/ml:
| | |
|---|---|
| low-molecular hyaluronic acid and/or its pharmaceutically acceptable salt with molecular weight from 100 kDa to 400 kDa | 2-8 |
| sodium lactate | 0.5-3 |
| lactic acid | 0.1-1 |
| decamethoxine and/or its water-soluble salt | 0.05-0.5 |
| glycerol | 20-50 |
| hydroxypropyl methylcellulose | 15-50 |
| water | q.s. to 1 ml. |

57. The pharmaceutical composition according to claim 51, ***characterized in that*** the dosage form is a gel containing low-molecular hyaluronic acid and/or its pharmaceutically acceptable salt with molecular weight from 100 kDa to 400 kDa, decamethoxine and/or its water-soluble salt, sodium lactate, lactic acid and water, and containing as auxiliary pharmaceutical components glycerol and hydroxypropyl methylcellulose, at the following components ratio, mg/ml:
| | |
|---|---|
| low-molecular hyaluronic acid with molecular weight from 100 kDa to 400 kDa | 5 |
| sodium lactate | 1.42 |
| lactic acid | 0.36 |
| decamethoxine and/or its water-soluble salt | 0.2 |
| glycerol | 30 |
| hydroxypropyl methylcellulose | 30 |
| water | q.s. to 1 ml. |
